# EUROPEAN PATENT SPECIFICATION

(11) **EP 1 453 798 B1**
(45) Date of publication and mention of the grant of the patent: **15.10.2008**
(21) Application number: 02794233.3
(22) Date of filing: 12.12.2002
(51) Int. Cl.: C07C 401/00, A61K 31/59, A61P 11/06, A61P 17/06, A61P 35/00, A61P 29/00, A61P 37/00

(54) **(20S)-1ALPHA-HYDROXY-2-METHYLENE-19-NOR-BISHOMOPREGNACALCIFEROL AND ITS USES**
(20S)-1 ALPHA-HYDROXY-2-METHYLEN-19-NOR-BISHOMOPREGNACALCIFEROL UND IHRE VERWENDUNGEN
(20S)-1ALPHA-HYDROXY-2-METHYLENE-19-NOR-BISHOMOPREGNACALCIFEROL ET SES UTILISATIONS

(30) Priority: 13.12.2001 US 341138 P; 18.02.2002 US 78204
(43) Date of publication of application: 08.09.2004
(73) Proprietor: WISCONSIN ALUMNI RESEARCH FOUNDATION, Madison, WI 53705-7365 (US)
(72) Inventor: DELUCA, Hector, F., Deerfield, WI 53531 (US); PLUM, Lori, A., Madison, WI 53705 (US); CLAGETT-DAME, Margaret, Deerfield, WI 53531 (US); THODEN, James, B., Madison, WI 53711 (US); HOLDEN, Hazel, M., Fitchburg, WI 53711 (US); GOWLUGARI, Sumithra, Fremont, CA 94555 (US); GRZYWACZ, Pawel, Madison, WI 53703 (US)
(74) Representative: McCluskie, Gail Wilson
(86) International application number: PCT/US2002/039715
(87) International publication number: WO 2003/051828

(56) References cited:
- WO-A-01/74766
- WO-A-02/20021
- WO-A-86/06255
- WO-A-98/41501
- DATABASE CA [Online] CHEMICAL ABSTRACTS SERVICE, COLUMBUS, OHIO, US; OSTREM, VOULA K. ET AL: "Induction of monocytic differentiation of HL-60 cells by 1,25-dihydroxyvitamin D analogs" retrieved from STN Database accession no. 107:191147 XP002238137 & JOURNAL OF BIOLOGICAL CHEMISTRY (1987), 262(29), 14164-71 ,
- DATABASE CA [Online] CHEMICAL ABSTRACTS SERVICE, COLUMBUS, OHIO, US; DELUCA, HECTOR F. ET AL: "Analogs of the hormonal form of vitamin D and their possible use in leukemia" retrieved from STN Database accession no. 108:180270 XP002238138 & PROGRESS IN CLINICAL AND BIOLOGICAL RESEARCH (1988), 259(NUTR., GROWTH, CANCER), 41-55 ,
- DATABASE CA [Online] CHEMICAL ABSTRACTS SERVICE, COLUMBUS, OHIO, US; HEKUTAA, EFU DERUUKA ET AL: "Cell differentiation-inducing agents containing secosterols" retrieved from STN Database accession no. 120:153700 XP002238139 & JP 05 238937 A (WISCONSIN ALUMNI RES FOUND, JAPAN) 17 September 1993 (1993-09-17)

## Description

### BACKGROUND OF THE INVENTION

This invention relates to vitamin D compounds, and more particularly to (20S)-1α-hydroxy-2-methylene-19-nor-bishomopregnacalciferol and its pharmaceutical uses.

The natural hormone, 1α,25-dihydroxyvitamin D₃ and its analog in ergosterol series, i.e. 1α,25-dihydroxyvitamin D₂ are known to be highly potent regulators of calcium homeostasis in animals and humans, and their activity in cellular differentiation has also been established, Ostrem et al., Proc. Natl. Acad. Sci. USA, 84, 2610 (1987). Many structural analogs of these metabolites have been prepared and tested, including 1α-hydroxyvitamin D₃, 1α-hydroxyvitamin D₂, various side chain homologated vitamins and fluorinated analogs. Some of these compounds exhibit an interesting separation of activities in cell differentiation and calcium regulation. This difference in activity may be useful in the treatment of a variety of diseases as renal osteodystrophy, vitamin D-resistant rickets, osteoporosis, psoriasis, and certain malignancies.

Recently, a new class of vitamin D analogs has been discovered, i.e. the so called 19-nor-vitamin D compounds, which are characterized by the replacement of the A-ring exocyclic methylene group (carbon 19), typical of the vitamin D system, by two hydrogen atoms. Biological testing of such 19-nor-analogs (e.g., 1α,25-dihydroxy-19-nor-vitamin D₃) revealed a selective activity profile with high potency in inducing cellular differentiation, and very low calcium mobilizing activity. Thus, these compounds are potentially useful as therapeutic agents for the treatment of malignancies, or the treatment of various skin disorders. Two different methods of synthesis of such 19-nor-vitamin D analogs have been described (Perlman et al., Tetrahedron Lett. 31, 1823 (1990); Perlman et al., Tetrahedron Lett. 32, 7663 (1991), and DeLuca et al., U.S. Pat. No. 5,086,191).

In U.S. Pat. No. 4,666,634, 2β-hydroxy and alkoxy (e.g., ED-71) analogs of 1α,25-dihydroxyvitamin D₃ have been described and examined by Chugai group as potential drugs for osteoporosis and as antitumor agents. See also Okano et al., Biochem. Biophys. Res. Commun. 163, 1444 (1989). Other 2-substituted (with hydroxyalkyl, e.g., ED-120, and fluoroalkyl groups) A-ring analogs of 1α,25-dihydroxyvitamin D₃ have also been prepared and tested (Miyamoto et al., Chem. Pharm. Bull. 41, 1111 (1993); Nishii et al., Osteoporosis Int. Suppl. 1, 190 (1993); Posner et al., J. Org. Chem. 59, 7855 (1994), and J. Org. Chem. 60, 4617 (1995)).

Recently, 2-substituted analogs of 1α,25-dihydroxy-19-nor-vitamin D₃ have also been synthesized, i.e. compounds substituted at 2-position with hydroxy or alkoxy groups (DeLuca et al., U.S. Pat. No. 5,536,713), with 2-alkyl groups (DeLuca et al U.S. Patent No. 5,945,410), and with 2-alkylidene groups (DeLuca et al U.S. Patent No. 5,843,928), which exhibit interesting and selective activity profiles. All these studies indicate that binding sites in vitamin D receptors can accommodate different substituents at C-2 in the synthesized vitamin D analogs.

In a continuing effort to explore the 19-nor class of pharmacologically important vitamin D compounds, an analog which is characterized by the presence of a methylene substituent at the carbon 2 (C-2) has been synthesized and tested. Of particular interest is the analog which is characterized by a hydroxyl group at carbon 1 and a shortened side chain attached to carbon 20, i.e. (20S)-1α-hydroxy-2-methylene-19-nor-bishomopregnacalciferol (referred to herein as 2-MbisP). This vitamin D analog seemed an interesting target because the relatively small methylene group at C-2 should not interfere with binding to the vitamin D receptor. Moreover, molecular mechanics studies performed on the model 1α-hydroxy-2-methylene-19-nor-vitamins indicate that such molecular modification does not change substantially the conformation of the cyclohexanediol ring A. However, introduction of the 2-methylene group into 19-nor-vitamin D carbon skeleton changes the character of its 1α- and 3β- A-ring hydroxyls. They are both now in the allylic positions, similarly, as 1α-hydroxyl group (crucial for biological activity) in the molecule of the natural hormone, 1α,25-(OH)₂D₃.

The present invention also relates to the purification of 2-methylene-19-nor-20(S)-1α-hydroxy-bis-homo-pregnacalciferol (2-MbisP) by preparing it in crystalline form.

Purification of organic compounds, especially those designated for pharmaceutical use, is of considerable importance for chemists synthesizing such compounds. Preparation of the compound usually requires many synthetic steps and, therefore, the final product can be contaminated not only with side-products derived from the last synthetic step of the procedure but also with compounds that were formed in previous steps. Even chromatographic purification, which is a very efficient but relatively time-consuming process, does not usually provide compounds which are sufficiently pure to be used as drugs.

Depending on the method used to synthesize 1α-hydroxyvitamin D compounds, different minor undesirable compounds can accompany the final product. Thus, for example, if direct C-1 hydroxylation of 5,6-trans geometric isomer of vitamin D is performed, followed by SeO₂/NMO oxidation and photochemical irradiation [see Andrews et al., J. Org. Chem. 51, 1635 (1986); Calverley et al., Tetrahedron 43, 4609 (1987); Choudry et al., J. Org. Chem. 58, 1496 (1993)], the final 1α-hydroxyvitamin D product can be contaminated with 1β-hydroxy- as well as 5,6-trans isomers. If the method consists of C-1 allylic oxidation of the 4-phenyl-1,2,4-triazoline-3,5-dione adduct of the previtamin D compound, followed by cycloreversion of the modified adduct under basic conditions [Nevinckx et al., Tetrahedron 47, 9419 (1991); Vanmaele et al., Tetrahedron 41, 141 (1985) and 40, 1179 (1991); Vanmaele et al., Tetrahedron Lett. 23, 995 (1982)], one can expect that the desired 1α-hydroxyvitamin can be contaminated with the previtamin 5(10),6,8-triene and 1β-hydroxy isomer. One of the most useful C-1 hydroxylation methods, of very broad scope and numerous applications, is the experimentally simple procedure elaborated by Paaren et al. [see J. Org. Chem. 45, 3253 (1980) and Proc. Natl. Acad. Sci. U.S.A. 75, 2080 (1978)]. This method consists of allylic oxidation of 3,5-cyclovitamin D derivatives, readily obtained from the buffered solvolysis of vitamin D tosylates, with SeO₂/*t*-BuOOH and subsequent acid-catalyzed cycloreversion to the desired 1α-hydroxy compounds. Taking into account this synthetic path it is reasonable to assume that the final product can be contaminated with 1α-hydroxy epimer, 5,6-trans isomer and the previtamin D form. 1α-hydroxyvitamin D₄ is another undesirable contaminant found in 1α-hydroxyvitamin D compounds synthesized from vitamin D₂ or from ergosterol. 1α-hydroxyvitamin D₄ results from C-1 oxidation of vitamin D₄, which in turn is derived from contamination of the commercial ergosterol material. Typically, the final product may contain up to about 1.5% by weight 1α-hydroxyvitamin D₄. Thus, a purification technique that would eliminate or substantially reduce the amount of 1α-hydroxyvitamin D₄ in the fmal product to less than about 01.-0.2% would be highly desirable.

The vitamin D conjugated triene system is not only heat- and light-sensitive but it is also prone to oxidation, leading to the complex mixture of very polar compounds. Oxidation usually happens when a vitamin D compound has been stored for a prolonged time. Other types of processes that can lead to a partial decomposition of vitamin D compounds consist of the some water-elimination reactions; their driving force is allylic (1α-) and homoallylic (3β-) position of the hydroxy groups. The presence of such above-mentioned oxidation and elimination products can be easily detected by thin-layer chromatography. Thus, for example, using precoated aluminum silica sheets [with UV indicator; from EM Science (Cherry Hill, NJ)] and solvent system hexane-ethyl acetate (4:6), the spot of 1α-OH-D₂ (R_{f} 0.27) and its elimination products (R_{f}'s ca. 0.7-0.9) are visible in ultraviolet light. Also, after spraying with sulfuric acid and heating, an additional spot can be visualized (R_{f} 0), derived from oxidation products.

Usually, all 1α-hydroxylation procedures require at least one chromatographic purification. However, even chromatographically purified 1α-hydroxyvitamin D compounds, although showing consistent spectroscopic data, suggesting homogeneity, does not meet the purity criteria required for therapeutic agents that can be orally, parenterally or transdermally administered. Therefore, it was evident that a suitable method of purification of 2MBP is required.

### SUMMARY OF THE INVENTION

The present invention is directed toward (20S)-1α-hydroxy-2-methylene-19-nor-bishomopregnacalciferol (2-MbisP), its biological activity, various pharmaceutical uses for this compound, and a method of purifying the compound to obtain it in crystalline form.

Structurally this 19-nor analog is characterized by the general formula I shown below:

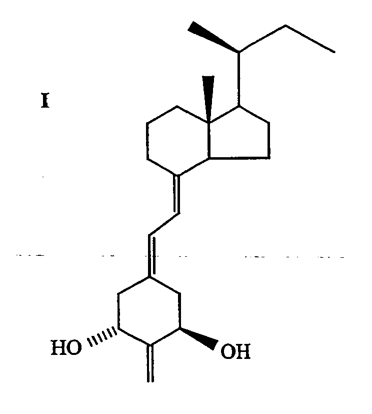

The above compound exhibits a desired, and highly advantageous, pattern of biological activity. This compound is characterized by relatively high binding to vitamin D receptors, but very low intestinal calcium transport activity, as compared to that of 1α,25-dihydroxyvitamin D₃, and has very low ability to mobilize calcium from bone, as compared to 1α,25-dihydroxyvitamin D₃. Hence, this compound can be characterized as having little, if any, calcemic activity. Thus, it may be useful as a therapy for suppression of secondary hyperparathyroidism of renal osteodystrophy.

The compound of the invention has also been discovered to be especially suited for treatment and prophylaxis of human disorders which are characterized by an imbalance in the immune system, e.g. in autoimmune diseases, including multiple sclerosis, lupis, diabetes mellitus, host versus graft reaction, and rejection of organ transplants; and additionally for the treatment of inflammatory diseases, such as rheumatoid arthritis, asthma, and inflammatory bowel diseases such as celiac disease and croans disease, as well as the improvement of bone fracture healing and improved bone grafts. Acne, alopecia and hypertension are other conditions which may be treated with the compound of the invention.

The above compound is also characterized by relatively high cell differentiation activity. Thus, this compound also provides a therapeutic agent for the treatment of psoriasis, or as an anti-cancer agent, especially against leukemia, colon cancer, breast cancer and prostate cancer. In addition, due to its relatively high cell differentiation activity, this compound provides a therapeutic agent for the treatment of various skin conditions including wrinkles, lack of adequate dermal hydration, i.e. dry skin, lack of adequate skin firmness, i.e. slack skin, and insufficient sebum secretion. Use of this compound thus not only results in moisturizing of skin but also improves the barrier function of skin.

The compound may be present in a composition to treat the above-noted diseases and disorders in an amount from about 0.01 µg/gm to about 100 µg/gm of the composition, and may be administered topically, transdermally, orally or parenterally in dosages of from about 0.01µg/day to about 100 µg/day.

Purification of 2-MbisP by means of crystallization was also investigated. The solvent plays a crucial role in a crystallization process, and is typically an individual liquid substance or a suitable mixture of different liquids. For crystallizing 2-MbisP, the most appropriate solvent and/or solvent system is
characterized by the following factors:
(1) low toxicity;
(2) low boiling point;
(3) significant dependence of solubility properties with regard to temperature (condition necessary for providing satisfactory crystallization yield); and
(4) relatively low cost.
   It was found that highly apolar solvents (e.g. hydrocarbons) were not suitable due to the low solubility of 2-MbisP in them. Quite the reverse situation occurred in highly polar solvent media (e.g. alcohols), in which 2-MbisP showed too high solubility. Therefore, it was concluded that for the successful crystallization of 2-MbisP, a solvent of medium polarity is required. Interestingly, hexane, so frequently used for crystallization purposes, was found less suitable for crystallization of 2-MbisP. However, it was found that an individual solvent, namely acetone, was most useful for the crystallization of 2-MbisP. In addition, it is believed that solvent systems utilizing ethyl acetate, or isopropanol, or chloroform, or dichloromethane, or diethyl ether would also perform well. These solvents are all very easy to remove by evaporation or other well known methods. In all cases the crystallization process occurred easily and efficiently; and the precipitated crystals were sufficiently large to assure their recovery by filtration.

### BRIEF DESCRIPTION OF THE DRAWINGS

Figures 1A-1E illustrate the structures of the compounds described and tested herein, namely, 1α,25-dihydroxy-19-nor-vitamin D₃, hereinafter 1,25(OH)₂19-nor-D₃ (Fig. 1A); the native hormone 1α,25-dihydroxyvitamin D₃, hereinafter 1,25(OH)₂D₃ (Fig. 1B); 1α-hydroxy-2-methylene-19-nor-pregnacalciferol, hereinafter 2-Mpregna (Fig. 1C); (20S)-1α-hydroxy-2-methylene-19-nor-bishomopregnacalciferol, hereinafter 2-MbisP (Fig. 1D); and 1α-hydroxy-2-methylene-19-nor-homopregnacalciferol, hereinafter 2-MP (Fig. 1E);
Figure 2 is a graph illustrating the relative activity of 1,25(OH)₂ 19-nor-D_{3,} 2-Mpregna, 2-MbisP, 2-MP and 1,25(OH)₂D₃ to compete for binding with [³H]-1,25-(OH)₂-D₃ to the full-length recombinant rat vitamin D receptor;
Figure 3 is a graph illustrating the percent HL-60 cell differentiation as a function of the concentration of 1,25(OH)₂ 19-nor-D₃, 2-Mpregna, 2-MbisP, 2-MP and 1,25(OH)₂D₃;
Figures 4A-4C are bar graphs illustrating the bone calcium mobilization activity of 1,25(OH)₂D₃ and 1,25(OH)₂ 19-nor-D₃ as compared to 2-MbisP (Fig. 4A), 2-Mpregna (Fig. 4B), and 2-MP (Fig. 4C);
Figures 5A-5C are bar graphs illustrating the intestinal calcium transport activity of 1,25(OH)₂D₃ and 1,25(OH)₂ 19-nor-D₃ as compared to 2-MbisP (Fig. 4A), 2-Mpregna (Fig. 4B), and 2-MP (Fig. 4C);
Figure 6 is a bar graph illustrating blood serum calcium levels in female rats after treatment with chronic doses of 1,25(OH)₂D₃ and 1,25(OH)₂ 19-nor-D₃ as compared to 2-MbisP (Fig. 6A), 2-Mpregna (Fig. 6B), and 2-MP (Fig. 6C); and
Figure 7 is an illustration of the of the three dimensional structure of 2-MbisP as defined by the atomic positional parameters discovered and set forth herein.

### DETAILED DESCRIPTION OF THE INVENTION

(20S)-1α-hydroxy-2-methylene-19-nor-bishomopregnacalciferol (referred to herein as 2-MbisP) was synthesized and tested. Structurally, this 19-nor analog is characterized by the general formula I previously illustrated herein.

The preparation of (20S)-1α-hydroxy-2-methylene-19-norbishomopregnacalciferol having the basic structure **I** can be accomplished by a common general method, i.e. the condensation of a bicyclic Windaus-Grundmann type ketone **II** with the allylic phosphine oxide **III** to the corresponding 2-methylene-19-nor-vitamin D analog **IV** followed by deprotection at C-1 and C-3 in the latter compound **IV** to obtain compound **I,** i.e. 2-MbisP. 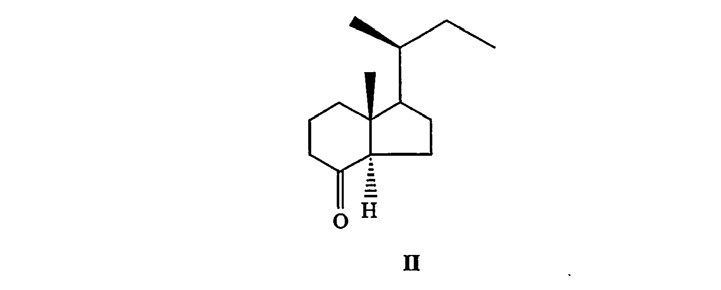 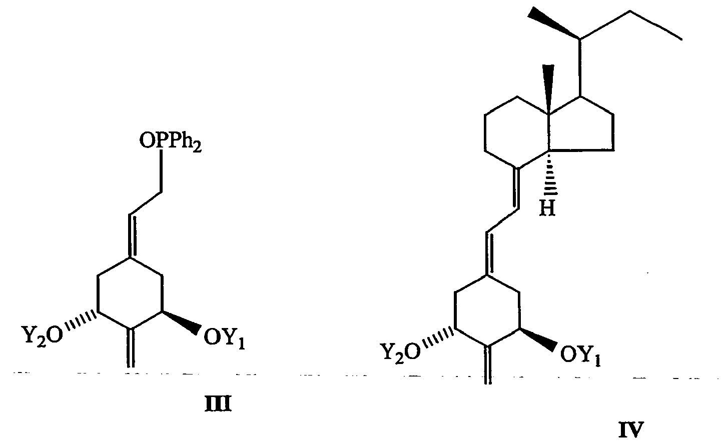 In the structures **II, III,** and **IV** groups Y₁ and Y₂ are hydroxy-protecting groups, it being also understood that any functionalities that might be sensitive, or that interfere with the condensation reaction, be suitably protected as is well-known in the art. The process shown above represents an application of the convergent synthesis concept, which has been applied effectively for the preparation of vitamin D compounds [e.g. Lythgoe et al., J. Chem. Soc. Perkin Trans. I, 590 (1978); Lythgoe, Chem. Soc. Rev. 9, 449 (1983); Toh et al., J. Org. Chem. 48, 1414 (1983); Baggiolini et al., J. Org. Chem. 51, 3098 (1986); Sardina et al., J. Org. Chem. 51, 1264 (1986); J. Org. Chem. 51, 1269 (1986); DeLuca et al., U.S. Pat. No. 5,086,191; DeLuca et al., U.S. Pat. No. 5,536,713].

Hydrindanones of the general structure **II** are known, or can be prepared by known methods.

For the preparation of the required phosphine oxides of general structure **III,** a synthetic route has been developed starting from a methyl quinicate derivative which is easily obtained from commercial (1R,3R,4S,5R)-(-)-quinic acid as described by Perlman et al., Tetrahedron Lett. 32, 7663 (1991) and DeLuca et al., U.S. Pat. No. 5,086,191.

The overall process of the synthesis of compound I is illustrated and described more completely in U.S. Patent No. 5,843,928 entitled "2-Alkylidene-19-Nor-Vitamin D Compounds" and in U.S. Patent No. 6,440,953 entitled "1α-Hydroxy-2-Methylene-19-Nor-Homopregnacalciferol and its Uses."

As used in the description and in the claims, the term "hydroxy-protecting group" signifies any group commonly used for the temporary protection of hydroxy functions, such as for example, alkoxycarbonyl, acyl, alkylsilyl or alkylarylsilyl groups (hereinafter referred to simply as "silyl" groups), and alkoxyalkyl groups. Alkoxycarbonyl protecting groups are alkyl-O-CO- groupings such as methoxycarbonyl, ethoxycarbonyl, propoxycarbonyl, isopropoxycarbonyl, butoxycarbonyl, isobutoxycarbonyl, tert-butoxycarbonyl, benzyloxycarbonyl or allyloxycarbonyl. The term "acyl" signifies an alkanoyl group of 1 to 6 carbons, in all of its isomeric forms, or a carboxyalkanoyl group of 1 to 6 carbons, such as an oxalyl, malonyl, succinyl, glutaryl group, or an aromatic acyl group such as benzoyl, or a halo, nitro or alkyl substituted benzoyl group. The word "alkyl" as used in the description of the claims, denotes a straight-chain or branched alkyl radical of 1 to 10 carbons, in all its isomeric forms. Alkoxyalkyl protecting groups are groupings such as methoxymethyl, ethoxymethyl, methoxyethoxymethyl, or tetrahydrofuranyl and tetrahydropyranyl. Preferred silyl-protecting groups are trimethylsilyl, triethylsilyl, t-butyldimethylsilyl, dibutylmethylsilyl, diphenylmethylsilyl, phenyldimethylsilyl, diphenyl-t-butylsilyl and analogous alkylated silyl radicals. The term "aryl" specifies a phenyl-, or an alkyl-, nitro- or halo-substituted phenyl group.

A "protected hydroxy" group is a hydroxy group derivatised or protected by any of the above groups commonly used for the temporary or permanent protection of hydroxy functions, e.g. the silyl, alkoxyalkyl, acyl or alkoxycarbonyl groups, as previously defined. The terms "hydroxyalkyl", "deuteroalkyl", "aminoalkyl", "halogenalkyl", "alkoxyalkyl", "aryloxyalkyl", and "fluoroalkyl" refer to an alkyl radical substituted by one or more hydroxy, deuterium, amino, halogen, alkoxy, aryloxy, or fluoro group respectively. A "halogen" group includes any of the five elements fluorine, chlorine, bromine, iodine and astatine that form a part of group VIIA of the periodic table.

Referring now to Schemes I-III, there are illustrated three different methods of synthesizing 2-MbisP starting with vitamin D₂. The first five steps are identical (shown in Scheme I) for each synthesis to obtain the protected 20(S)-aldehyde. Thereafter, in Schemes I and II the aldehyde is converted to a protected 20(S)-alcohol which in turn has its side chain converted to bis-homo in two different ways (Scheme I versus Scheme II). In contrast, Scheme III illustrates a direct conversion of the protected 20(S)-aldehyde to the bis-homo side chain.

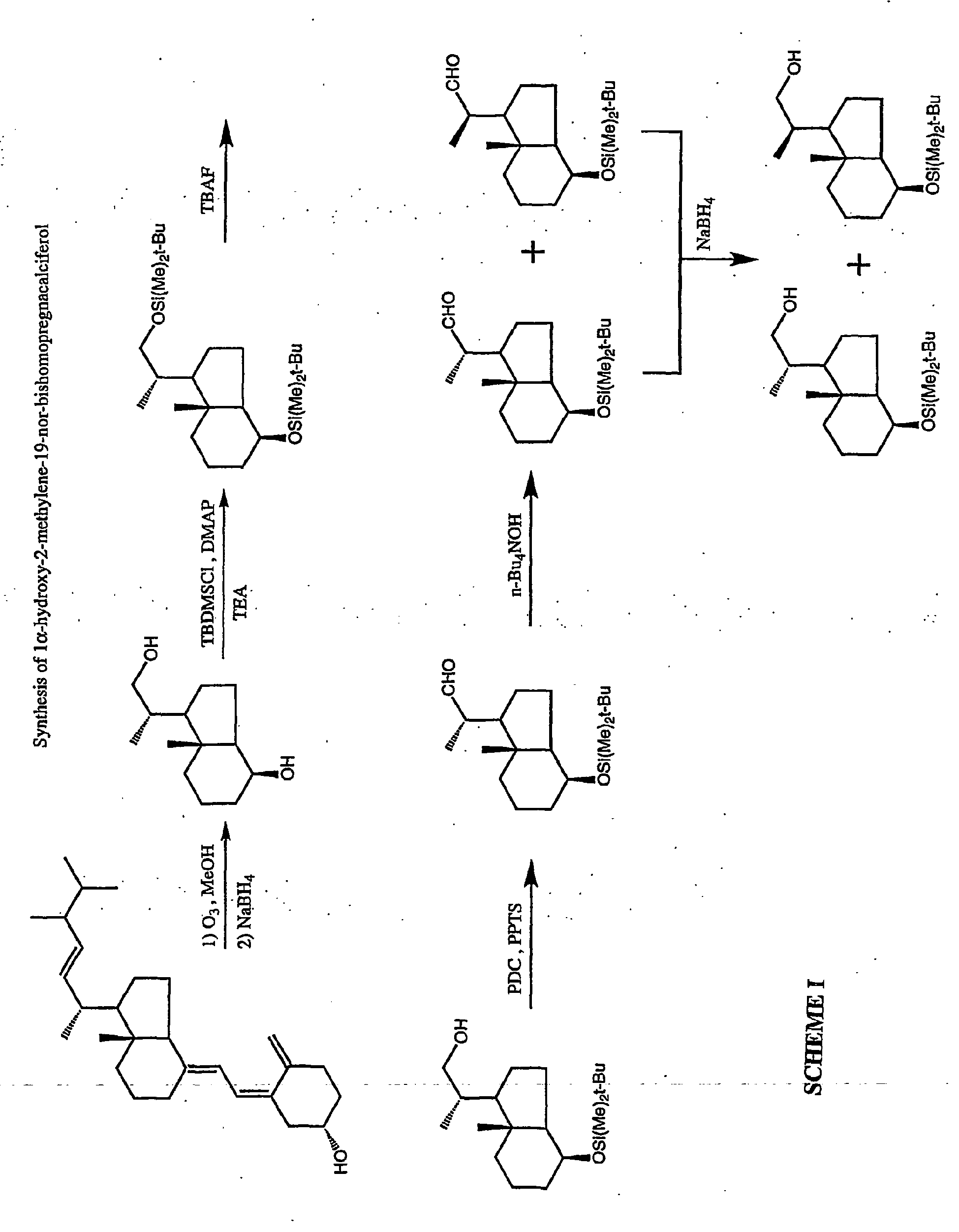

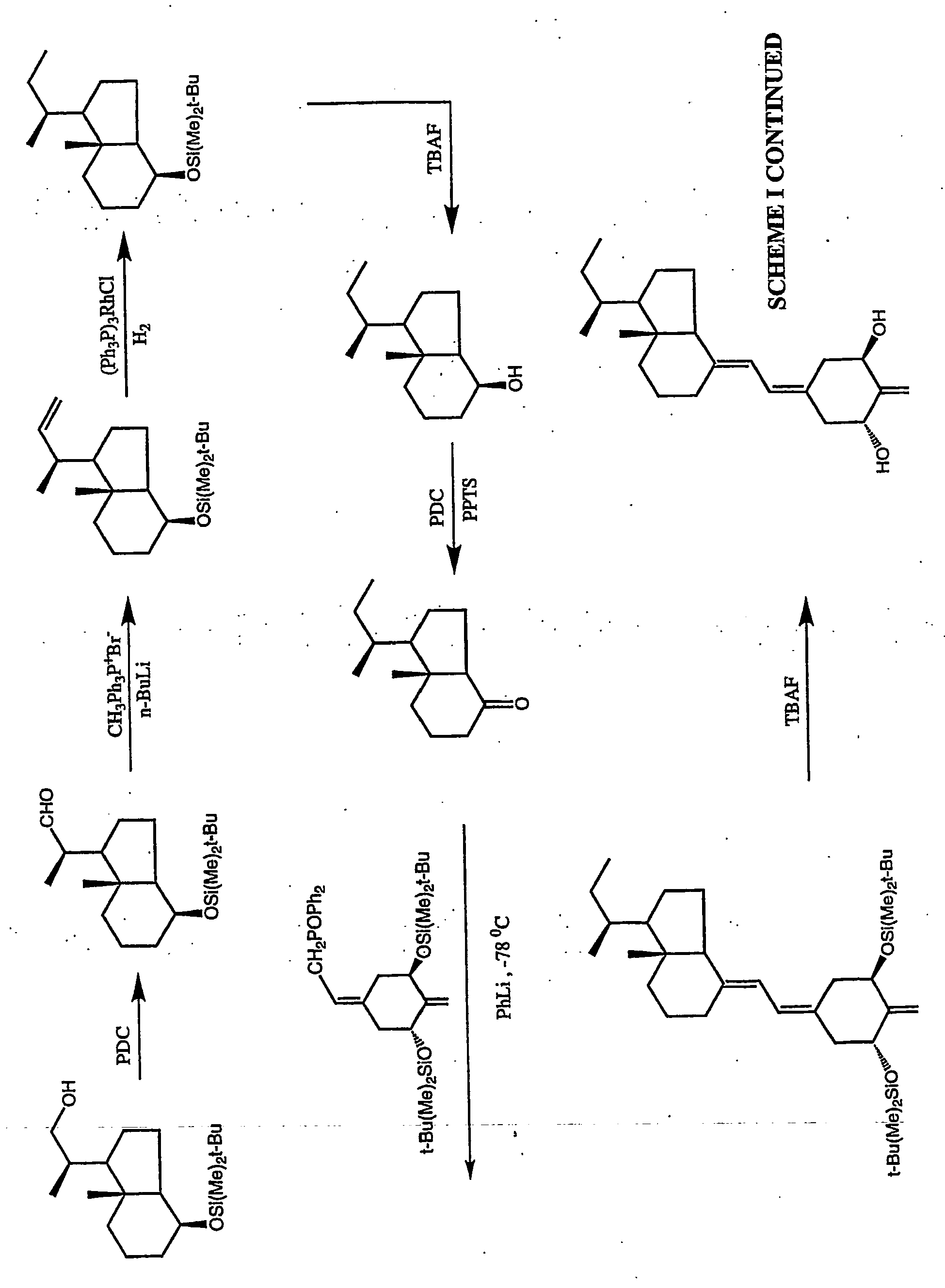

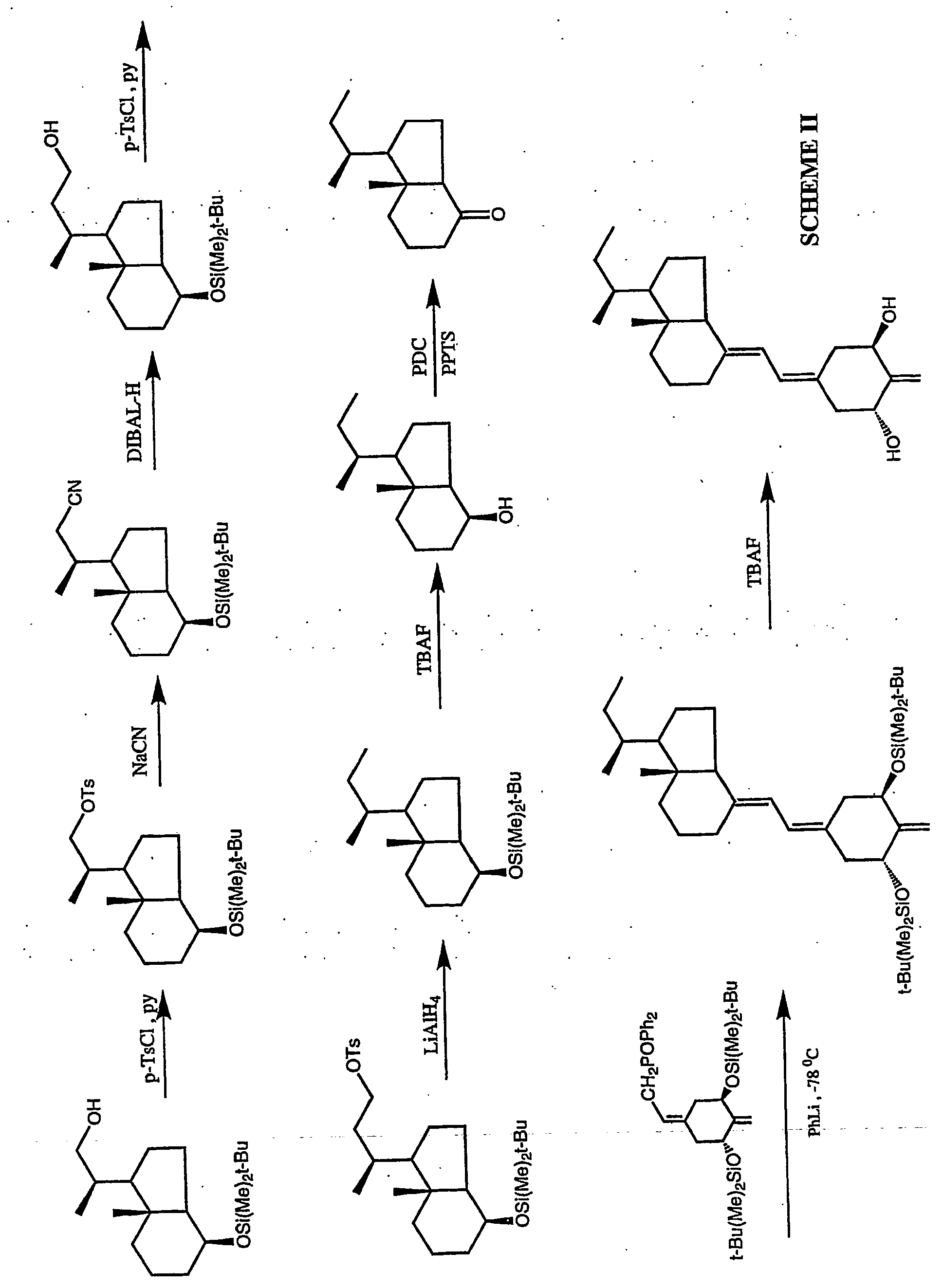

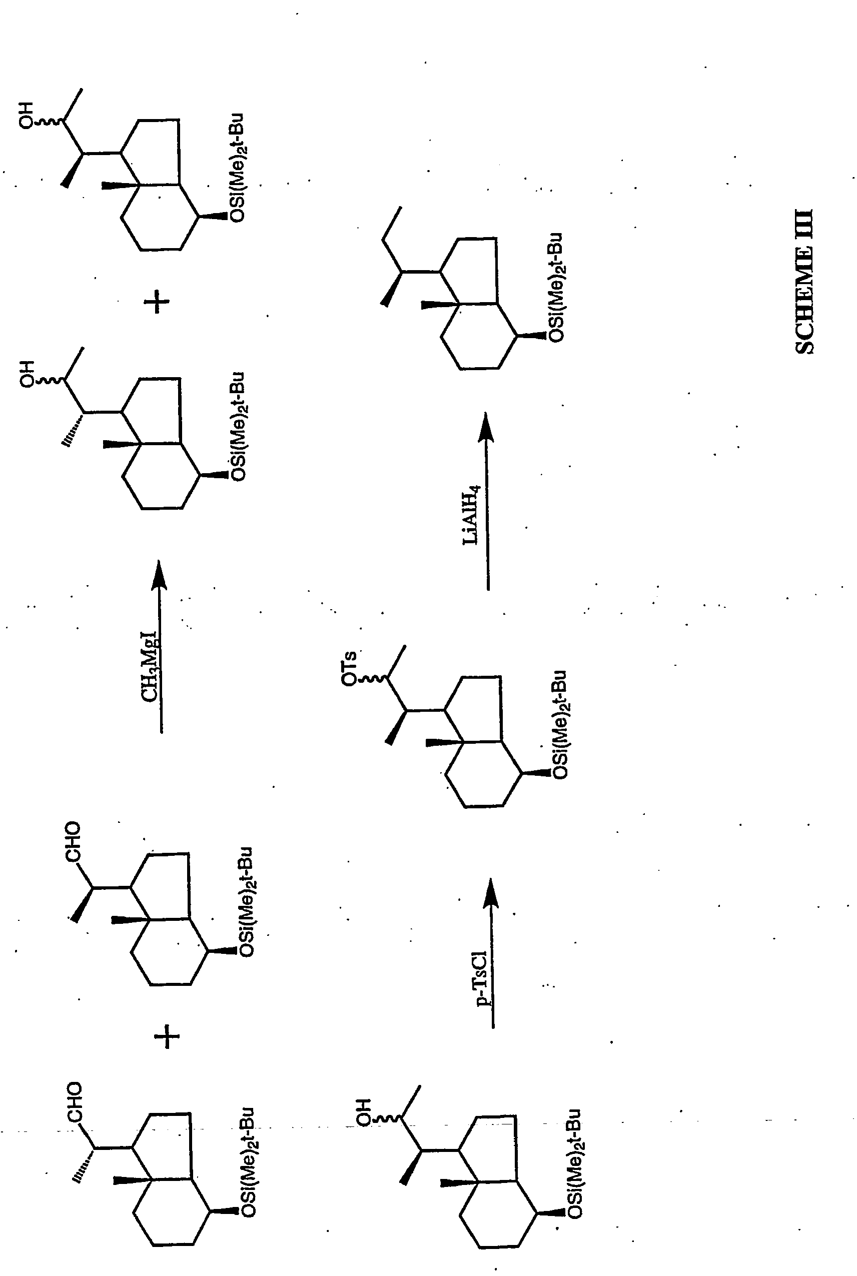

### BIOLOGICAL ACTIVITY OF (20S)-1α-HYDROXY-2-METHYLENE-19-NOR-BISHOMOPREGNACALCIFEROL

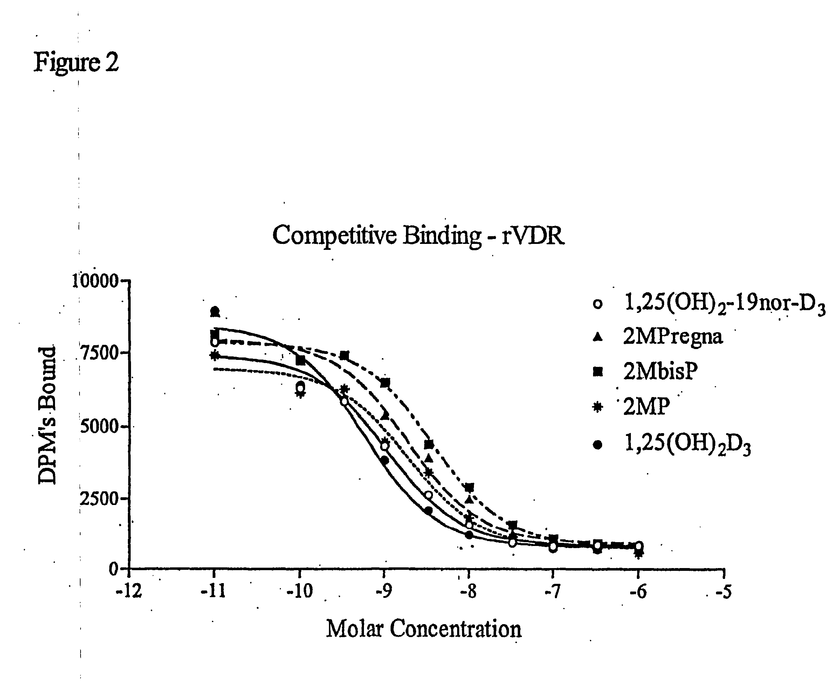

The introduction of a methylene group to the 2-position of 1α-hydroxy-19-nor-pregnacalciferol had little or no effect on binding to the porcine intestinal vitamin D receptor, as compared to 1α,25-dihydroxyvitamin D₃. This compound bound equally well to the porcine receptor as compared to the standard 1,25-(OH)₂D₃ (Figure 2). It might be expected from these results that this compound would have equivalent biological activity. Surprisingly, however, the 2-methylene substitution in 2-MbisP produced a highly selective analog with unique biological activity.

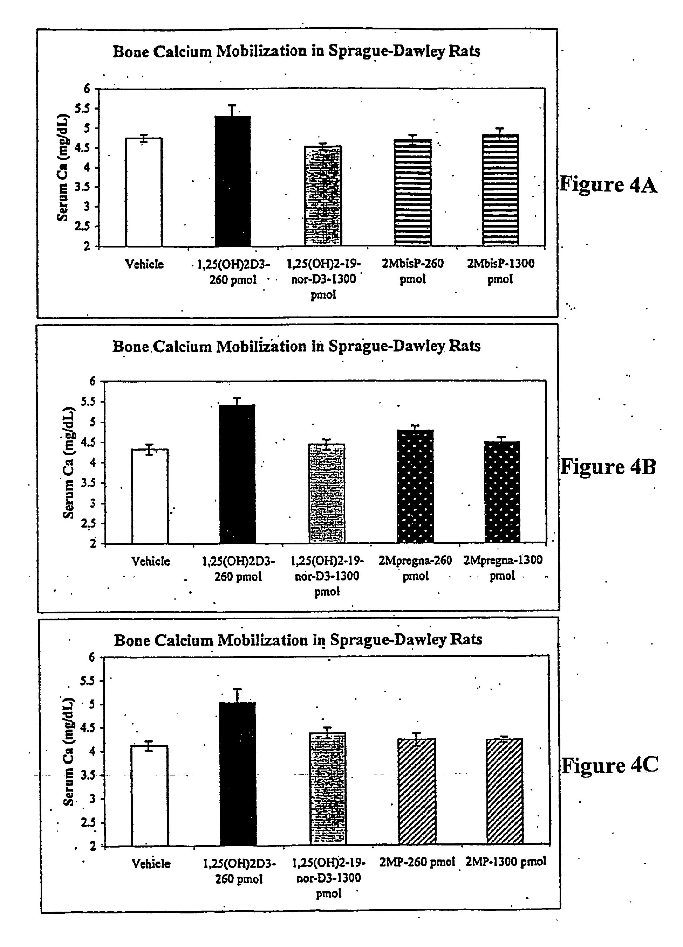

Figure 4B shows that 2- MbisP has very little activity as compared to that of 1,25-dihydroxyvitamin D₃ (1,25(OH)₂D₃), the natural hormone, in stimulating intestinal calcium transport.

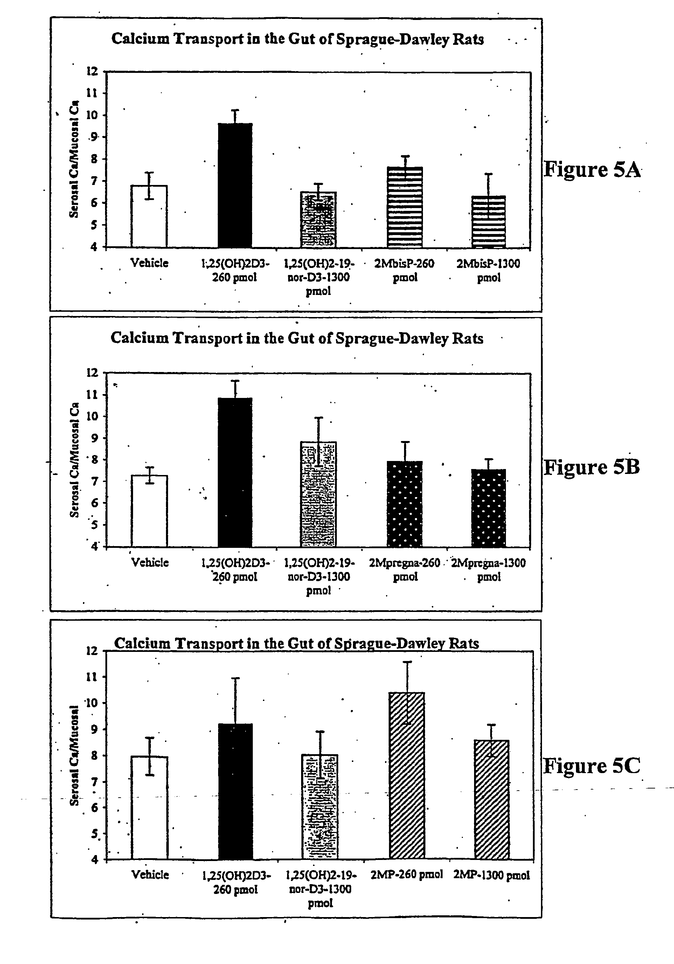

Figure 5B demonstrates that 2- MbisP has very little bone calcium mobilization activity, as compared to 1,25(OH)₂D₃.

Figures 4B and 5B thus illustrate that 2- MbisP may be characterized as having little, if any, calcemic activity.

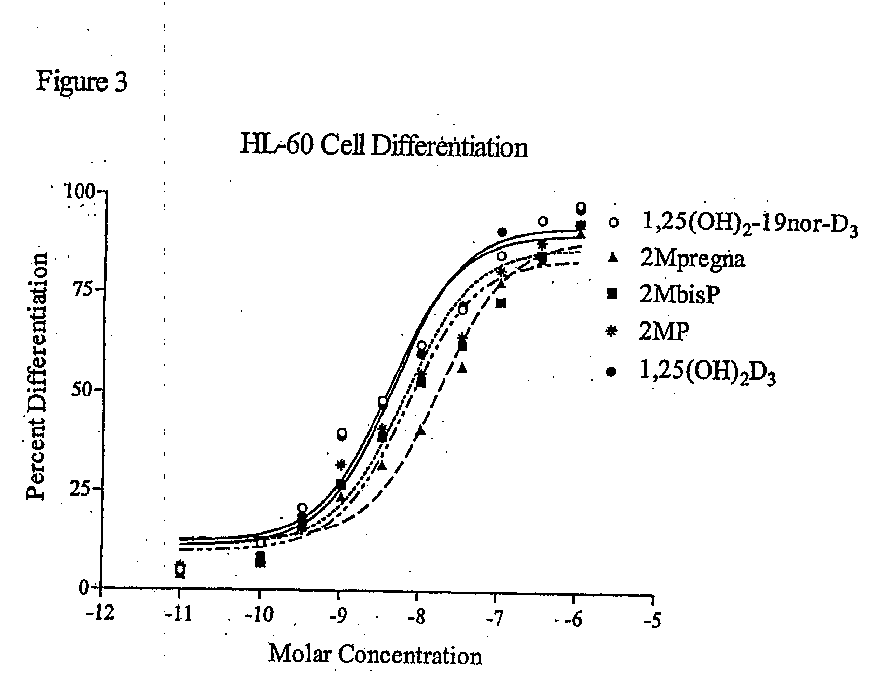

Figure 3 illustrates that 2- MbisP is almost as potent as 1,25(OH)₂D₃ on HL-60 differentiation, making it an excellent candidate for the treatment of psoriasis and cancer, especially against leukemia, colon cancer, breast cancer and prostate cancer. In addition, due to its relatively high cell differentiation activity, this compound provides a therapeutic agent for the treatment of various skin conditions including wrinkles, lack of adequate dermal hydration, i.e. dry skin, lack of adequate skin firmness, i.e, slack skin, and insufficient sebum secretion. Use of this compound thus not only results in moisturizing of skin but also improves the barrier function of skin.

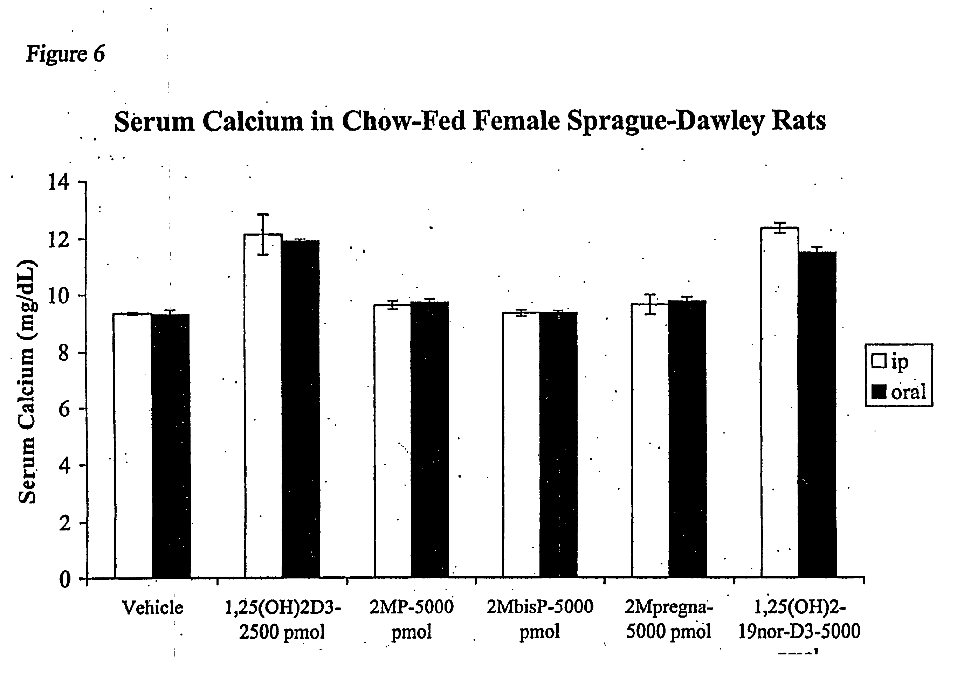

Figure 6 shows an analysis of serum calcium in rats after administration of chronic doses of 2- MbisP. These data provide further support for the data in Figures 4B and 5B that 2- MbisP has very little calcemic activity and thus a relatively low risk of producing hypercalcemia at recommended doses.

The battery of in vitro and in vivo assays described in Sicinski et al (J. Med. Chem. 41, 4662-4674, 1998) were used to compare the biological activities of the compounds, 2-Mpregna, 2-MbisP and 2-MP, with 1,25(OH)₂ 19-nor-D₃, and 1,25(OH)₂D₃, the native vitamin D hormone.

The differentiation of HL-60 promyelocytic into monocytes was determined as described by Ostrem et al (J. Biol. Chem. 262, 14164-14171, 1987).

### INTERPRETATION OF DATA

VDR binding and HL60 cell differentiation. 2-MbisP, 2-Mpregna and 2-MP are nearly equivalent (Ki=0.3, 0.6 and 0.3 nM for 2-Mpregna, 2-MbisP and 2-MP, respectively) in their ability to compete with [³H]-1,25(OH)₂D₃ for binding to the full-length recombinant rat vitamin D receptor (Figure 2). Furthermore, the competition binding activity of these three compounds is similar to that of 1,25(OH)₂ 19-nor-D₃ (Ki=0.2 nM), as well as the native hormone, 1,25(OH)₂D₃ (Ki=0.1 nM). There is little difference between any of these five compounds in their ability (efficacy or potency) to promote HL60 differentiation with the possible exception of 2-Mpregna (EC₅₀=17 nM), which is slightly less potent than 2-MbisP (EC₅₀=7 nM), 2-MP (EC₅₀=6 nM), 1,25(OH)₂ 19-nor-D₃ (EC₅₀=4 nM) and 1,25(OH)₂D₃ (EC₅₀=5 nM) (See Figure 3). This result suggests that 2- MbisP will be very effective in psoriasis because it has direct cellular activity in causing cell differentiation and in suppressing cell growth. It also indicates that it will have significant activity as an anti-cancer agent, especially against leukemia, colon cancer, breast cancer and prostate cancer, as well as against skin conditions such as dry skin (lack of dermal hydration), undue skin slackness (insufficient skin firmness), insufficient sebum secretion and wrinkles.

Calcium mobilization from bone and intestinal calcium absorption in vitamin D-deficient animals. Using vitamin D-deficient rats on a low calcium diet (0.02%), the activities of these compounds in intestine and bone were tested. As expected, the native hormone (1,25(OH)₂D₃) increased serum calcium levels at all dosages (Fig. 4). Figure 4 also shows that 2-MbisP, 2-Mpregna and 2-MP have little, if any, activity in mobilizing calcium from bone. Administration of 2-MbisP, 2-Mpregna, 2-MP or 1,25(OH)₂ 19-nor-D₃ at 260 pmol/day for 7 days did not result in mobilization of bone calcium, and increasing the amount of 2-MbisP, 2-Mpregna and 2-MP to 1300 pmol/day (5-fold) was without effect. Similar findings were obtained with 1,25(OH)₂ 19-nor-D₃.

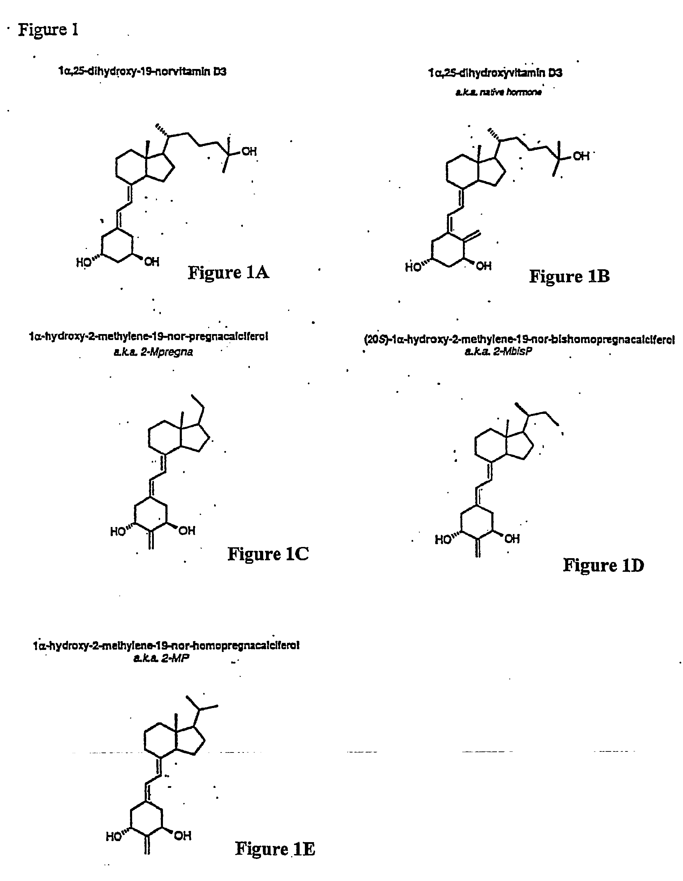

Intestinal calcium transport was evaluated in the same groups of animals using the everted gut sac method (Figure 5). These results show that 2-MbisP and 2-Mpregna do not promote intestinal calcium transport when administered at either 260 or 1300 pmol/day, whereas 1,25(OH)₂D₃ promotes a significant increase at the 260 pmol/day dose. In contrast to 2-MbisP and 2-Mpregna, 2-MP showed calcium transport activity equivalent to 1,25(OH)₂D₃ at this and the 5-fold higher concentration but higher doses actually decreased this activity. As shown in Figure 4, 1,25(OH)₂ 19-nor-D₃, like the 2-MbisP and 2-Mpregna derivatives, is devoid of intestinal calcium transport activity.

Serum calcium response in vitamin D-sufficient animals on a normal calcium diet. The desirability of 2-MbisP, 2-Mpregna and 2-MP is exemplified by their inability to produce hypercalcemia in normal animals compared to 1,25(OH)₂ 19-nor-D₃, and 1,25(OH)₂D₃. In the experiment shown in Figure 6, normal, female rats were given 2500 pmol/day for 7 days of 1,25(OH)₂D₃, or 5000 pmol/day of 2-MbisP, 2-Mpregna, 2-MP or 1,25(OH)₂ 19-nor-D₃. The doses were administered to two separate groups of animals by either the oral or intraperitoneal route, and serum calcium levels were assessed 4 hours after the last dose. Animals receiving 1,25(OH)₂D₃ by either route exhibited hypercalcemia, some severe enough to require euthanasia. Likewise, 1,25(OH)₂ 19-nor-D₃ produced frank hypercalcemia. In contrast, no increase in serum calcium was seen in any of the animals receiving the compounds, 2-MP, 2-MbisP or 2-Mpregna.

These results illustrate that 2-MbisP is an excellent candidate for numerous human therapies and that it may be useful in a number of circumstances such as autoimmune diseases, cancer, and psoriasis. 2-MbisP is an excellent candidate for treating psoriasis because: (1) it has significant VDR binding and cellular differentiation activity; (2) it is devoid of hypercalcemic liability unlike 1,25(OF)₂ 19-nor-D₃ and 1,25(OH)₂D₃; and (3) it is easily synthesized. Since 2-MbisP has significant binding activity to the vitamin D receptor, but has little ability to raise blood serum calcium, it may also be useful for the treatment of renal osteodystrophy.

For treatment purposes, the compound of this invention defined by formula **I** may be formulated for pharmaceutical applications as a solution in innocuous solvents, or as an emulsion, suspension or dispersion in suitable solvents or carriers, or as pills, tablets or capsules, together with solid carriers, according to conventional methods known in the art. Any such formulations may also contain other pharmaceutically-acceptable and non-toxic excipients such as stabilizers, anti-oxidants, binders, coloring agents or emulsifying or taste-modifying agents.

The compound may be administered orally, topically, parenterally or transdermally. The compound is advantageously administered by injection or by intravenous infusion or suitable sterile solutions, or in the form of liquid or solid doses via the alimentary canal, or in the form of creams, ointments, patches, or similar vehicles suitable for transdermal applications. Doses of from 0.01µg to 100 µg per day of the compounds are appropriate for treatment purposes, such doses being adjusted according to the disease to be treated, its severity and the response of the subject as is well understood in the art. Since the compound exhibits specificity of action, each may be suitably administered alone, or together with graded doses of another active vitamin D compound -- e.g. 1α-hydroxyvitamin D₂ or D₃, or 1α,25-dihydroxyvitamin D₃ -- in situations where different degrees of bone mineral mobilization and calcium transport stimulation is found to be advantageous.

Compositions for use in the above-mentioned treatments comprise an effective amount of the (20S)-1α-hydroxy-2-methylene-19-nor-bishomopregnacalciferol as defined by the above formula I as the active ingredient, and a suitable carrier. An effective amount of such compound for use in accordance with this invention is from about 0.01 µg to about 100 µg per gm of composition, and may be administered topically, transdermally, orally or parenterally in dosages of from about 0.01µg/day to about 100 µg/day.

The compound may be formulated as creams, lotions, ointments, topical patches, pills, capsules or tablets, or in liquid form as solutions, emulsions, dispersions, or suspensions in pharmaceutically innocuous and acceptable solvent or oils, and such preparations may contain in addition other pharmaceutically innocuous or beneficial components, such as stabilizers, antioxidants, emulsifiers, coloring agents, binders or taste-modifying agents.

The compound is advantageously administered in amounts sufficient to effect the differentiation of promyelocytes to normal macrophages. Dosages as described above are suitable, it being understood that the amounts given are to be adjusted in accordance with the severity of the disease, and the condition and response of the subject as is well understood in the art.

The formulations of the present invention comprise an active ingredient in association with a pharmaceutically acceptable carrier therefore and optionally other therapeutic ingredients. The carrier must be "acceptable" in the sense of being compatible with the other ingredients of the formulations and not deleterious to the recipient thereof.

Formulations of the present invention suitable for oral administration may be in the form of discrete units as capsules, sachets, tablets or lozenges, each containing a predetermined amount of the active ingredient; in the form of a powder or granules; in the form of a solution or a suspension in an aqueous liquid or non-aqueous liquid; or in the form of an oil-in-water emulsion or a water-in-oil emulsion.

Formulations for rectal administration may be in the form of a suppository incorporating the active ingredient and carrier such as cocoa butter, or in the form of an enema.

Formulations suitable for parenteral administration conveniently comprise a sterile oily or aqueous preparation of the active ingredient which is preferably isotonic with the blood of the recipient.

Formulations suitable for topical administration include liquid or semi-liquid preparations such as liniments, lotions, applicants, oil-in-water or water-in-oil emulsions such as creams, ointments or pastes; or solutions or suspensions such as drops; or as sprays.

For asthma treatment, inhalation of powder, self-propelling or spray formulations, dispensed with a spray can, a nebulizer or an atomizer can be used. The formulations, when dispensed, preferably have a particle size in the range of 10 to 100µ.

The formulations may conveniently be presented in dosage unit form and may be prepared by any of the methods well known in the art of pharmacy. By the term "dosage unit" is meant a unitary, i.e. a single dose which is capable of being administered to a patient as a physically and chemically stable unit dose comprising either the active ingredient as such or a mixture of it with solid or liquid pharmaceutical diluents or carriers.

### CRYSTALLIZATION OF 2-MbisP

The present invention also provides 2-methylene-19-nor-20(S)-1α-hydroxy-bis-homo-pregnacalciferol (2-MbisP) in crystalline form. The present invention further provides a valuable method of purification of 2-MbisP. The purification technique involves obtaining the 2-MbisP product in crystalline form by utilizing a crystallization procedure wherein the 2-MbisP material to be purified is dissolved using as the solvent a single solvent system comprised of acetone, ethyl acetate, isopropanol, chloroform, dichloromethane, or diethyl ether. The preferred solvent is acetone. Thereafter, the solvent can be removed by evaporation, with or without vacuum, or other means as is well known. The technique can be used to purify a wide range of final products containing 2-MbisP obtained from any known synthesis thereof, and in varying concentrations, i.e. from microgram amounts to kilogram amounts. As is well known to those skilled in this art, the amount of solvent utilized should be minimized and/or adjusted according to the amount of 2-MbisP to be purified.

The usefulness and advantages of the present crystallization procedures is shown in the following specific Examples 1 and 2. After crystallization, the precipitated material was observed under a microscope to confirm its crystalline form. Additionally, X-ray diffraction analysis was performed. Yields of crystallizations were high and the obtained crystals showed a relatively sharp melting point of 162-164°C.

The described crystallization process of the synthetic 2-MbisP product represents a valuable purification method, which can remove not only some side products derived from the synthetic path, but, moreover, concomitant 1α-hydroxyvitamin D₄. Such impurity is the result of the contamination of natural ergosterol with its 22,23-dihydro analog.

### Example 1

### Crystallization from acetone

(a) 2-MbisP product to be purified may be dissolved in boiling acetone (1.2 mL, Aldrich) under argon atmosphere, left at room temperature (68°F) for a few hours (1-3 hrs) and then in a refrigerator (35-45°F) overnight (8-12 hrs). The precipitated crystals should be filtered off, washed with a small volume of a cold (0°C) acetone and dried.
(b) These crystals of 2-MbisP may then be recrystallized with acetone (0.5 mL) as described in Example 1(a). The precipitated crystals have a relatively sharp melting point of 162-164°C, and were observed under a microscope to confirm their crystalline form.

### Example 2

### Experimental

A colorless plate-shaped crystal of dimensions 0.40 x 0.25 x 0.03 mm was utilized for the structural analysis. Intensity data were collected with a Siemens HISTAR area detector mounted on a platform goniometer using Cu Kα radiation (λ=1.54178 Å) focused with Goebel optics. The crystal was mounted on a thin nylon fiber with vacuum grease and data were collected at ambient temperature, 293°K. The intensity data were measured as a series of ω oscillation frames each of 0.25°; these were comprised of 5 scans (500 images each, 40 see./image) at detector 2θ of -85° and 2 scans (500 images each, 20 sec./image) at detector 20 of -40°. The detector was operated in 512 x 512 mode and was positioned 6.0 cm from the sample. Coverage of unique data was 95.4% complete to 58.88 degrees in θ. The first 50 frames were repeated at the end of data collection and showed essentially no decay of the crystal diffraction during the data collection. A total of 6917 data was measured in the range 4.38 < θ < 58.88°. The data were merged and scaled to form a set of 2697 independent data with R(int) = 0.0562 using SAINT (Bruker-AXS, Inc.).

The orthorhombic space group P2(1)2(1)2(1) was determined by systematic absences and statistical tests and verified by subsequent refinement. The structure was solved by direct methods and refined by full-matrix least-squares methods on *F*² using SHELXL-97 (G.M. Sheldrick, 1994; SHELXTL Version 5 Reference Manual, Bruker-AXS, Inc.). Hydrogen atom positions were intially located in Fourier difference maps, but refined by a riding model with idealized geometries. Non-hydrogen atoms were refined with anisotropic displacement parameters. A total of 229 parameters were refined against 0 restraints and 2697 data to give wR(*F*²) = 0.1369 and S =1.088 for weights ofw = 1/[s² (*F*²) + (0.0824 P)²], where P = [*F*ₒ² + 2*F*_{c}²]/3. The final R(F) was 0.0544 for the 2697 observed, [*F*> 2s(*F*)], data. The final difference map had maxima and minima of 0.126 and -0.153 e/Å³, respectively.

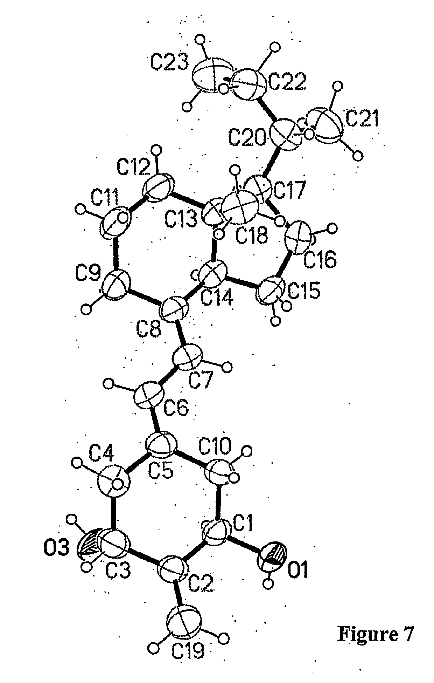

The three dimensional structure of 2-MbisP as defined by the following physical data and atomic positional parameters described and calculated herein is illustrated in Figure 7.

**Table 1. Crystal data and structure refinement for 2-MbisP.**

| | |
|---|---|
| Empirical formula | C23 H36 O2 |
| Formula weight | 344.52 |
| Temperature | 293(2) K |
| Wavelength | 1.54178 Å |
| Crystal system, space group | Orthorhombic, P2(1)2(1)2(1) |
| Unit cell dimensions | a = 6.6320(13) Å α = 90° |
| | b = 15.514(3) Å β = 90° |
| | c = 20.194(4) Å γ = 90° |
| Volume | 2077.7(7) Å³ |
| Z, Calculated density | 4, 1.101 Mg/m³ |
| Absorption coefficient | 0.520 mm⁻¹ |
| F(000) | 760 |
| Crystal size | 0.40 x 0.25 x 0.03 mm |
| Theta range for data collection | 4.38 to 58.88° |
| Limiting indices | -7<=h<=5, -12<=k<=17, -22<=1<=21 |
| Reflections collected / unique | 6917 / 2697 [Rᵢₙₜ= 0.0562] |
| Completeness to theta = 25.00 | 95.4 % |
| Refinement method | Full-matrix least-squares on F² |
| Data / restraints / parameters | 2697 / 0 / 229 |
| Goodness-of-fit on F² | 1.088 |
| Final R indices [I>2sigma(I)] | R1 = 0.0544, wR2 = 0.1369 |
| R indices (all data) | R1 = 0.0622, wR2 = 0.1453 |
| Largest diff. peak and hole | 0.126 and -0.153 e/A⁻³ |

**Table 2. Atomic coordinates (x 10⁴) and equivalent isotropic displacement parameters (Å² x 10³) for 2-MbisP. U(eq) is defined as one third of the trace of the orthogonalized Uij tensor.**

| | x | y | z | U(eq) |
|---|---|---|---|---|
| O(1) | 1842(3) | 3525(1) | 5011(1) | 62(1) |
| O(3) | 7954(4) | 3113(1) | 4476(2) | 78(1) |
| C(1) | 3883(5) | 3729(2) | 4852(2) | 50(1) |
| C(2) | 4569(5) | 3359(2) | 4201(2) | 58(1) |
| C(3) | 6698(5) | 3569(2) | 4030(2) | 61(1) |
| C(4) | 7074(6) | 4537(2) | 4057(2) | 59(1) |
| C(5) | 6301(5) | 4937(2) | 4686(2) | 48(1) |
| C(6) | 7497(5) | 5433(2) | 5059(2) | 52(1) |
| C(7) | 7012(5) | 5872(2) | 5673(2) | 53(1) |
| C(8) | 8248(5) | 6371(2) | 6020(2) | 50(1) |
| C(9) | 10390(5) | 6589(2) | 5828(2) | 65(1) |
| C(10) | 4161(5) | 4704(2) | 851(2) | 51(1) |
| C(11) | 10739(6) | 7562(2) | 5809(2) | 70(1) |
| C(12) | 10108(6) | 8006(2) | 6452(2) | 61(1) |
| C(13) | 7933(5) | 7806(2) | 6640(1) | 47(1) |
| C(14) | 7696(5) | 6814(2) | 6661(2) | 50(1) |
| C(15) | 5663(6) | 6668(2) | 6982(2) | 62(1) |
| C(16) | 5453(5) | 7416(2) | 7474(2) | 59(1) |
| C(17) | 7251(5) | 8038(2) | 7353(2) | 51(1) |
| C(18) | 6466(6) | 8205(2) | 6135(2) | 64(1) |
| C(19) | 3405(7) | 2898(3) | 3811(2) | 97(2) |
| C(20) | 6703(6) | 8967(2) | 7542(2) | 65(1) |
| C(21) | 6012(8) | 9017(3) | 8269(2) | 92(2) |
| C(22) | 8368(7) | 9636(2) | 7418(2) | 89(1) |
| C(23) | 10284(9) | 9512(3) | 7809(3) | 116(2) |

**Table 3. Bond lengths [Å] and angles [°] for 2-MbisP.**

| | | | |
|---|---|---|---|
| O(1)-C(1) | 1.426(4) | O(3)-C(3) | 1.416(4) |
| C(1)-C(2) | 1.505(5) | C(1)-C(10) | 1.524(4) |
| C(2)-C(19) | 1.314(5) | C(2)-C(3) | 1.490(5) |
| C(3)-C(4) | 1.522(4) | C(4)-C(5) | 1.504(5) |
| C(5)-C(6) | 1.335(4) | C(5)-C(10) | 1.502(5) |
| C(6)-C(7) | 1.452(4) | C(7)-C(8) | 1.327(4) |
| C(8)-C(9) | 1.511(5) | C(8)-C(14) | 1.511(4) |
| C(9)-C(11) | 1.527(5) | C(11)-C(12) | 1.530(5) |
| C(12)-C(13) | 1.523(5) | C(13)-C(18) | 1.539(5) |
| C(13)-C(14) | 1.548(4) | C(13)-C(17) | 1.553(4) |
| C(14)-C(15) | 1.513(5) | C(15)-C(16) | 1.534(5) |
| C(16)-C(17) | 1.553(5) | C(17)-C(20) | 1.535(5) |
| C(20)-C(22) | 1.536(6) | C(20)-C(21) | 1.540(5) |
| C(22)-C(23) | 1.508(7) | | |
| O(1)-C(1)-C(2) | 113.5(2) | O(1)-C(1)-C(10) | 109.6(2) |
| C(2)-C(1)-C(10) | 109.9(3) | C(19)-C(2)-C(3) | 122.5(3) |
| C(19)-C(2)-C(1) | 123.6(3) | C(3)-C(2)-C(1) | 113.9(3) |
| O(3)-C(3)-C(2) | 107.5(3) | O(3)-C(3)-C(4) | 111.9(3) |
| C(2)-C(3)-C(4) | 111.3(3) | C(5)-C(4)-C(3) | 112.5(3) |
| C(6)-C(5)-C(10) | 125.1(3) | C(6)-C(5)-C(4) | 120.7(3) |
| C(10)-C(5)-C(4) | 114.2(3) | C(5)-C(6)-C(7) | 128.3(3) |
| C(8)-C(7)-C(6) | 126.0(3) | C(7)-C(8)-C(9) | 125.1(3) |
| C(7)-C(8)-C(14) | 124.6(3) | C(9)-C(8)-C(14) | 110.3(3) |
| C(8)-C(9)-C(11) | 111.7(3) | C(5)-C(10)-C(1) | 110.8(2) |
| C(9)-C(11)-C(12) | 112.5(3) | C(13)-C(12)-C(11) | 112.2(3) |
| C(12)-C(13)-C(18) | 110.6(3) | C(12)-C(13)-C(14) | 107.8(3) |
| C(18)-C(13)-C(14) | 110.8(3) | C(12)-C(13)-C(17) | 117.3(3) |
| C(18)-C(13)-C(17) | 109.7(3) | C(14)-C(13)-C(17) | 100.1(2) |
| C(15)-C(14)-C(8) | 121.0(3) | C(15)-C(14)-C(13) | 104.6(3) |
| C(8)-C(14)-C(13) | 113.8(2) | C(14)-C(15)-C(16) | 104.2(3) |
| C(15)-C(16)-C(17) | 107.4(3) | C(20)-C(17)-C(16) | 111.3(3) |
| C(20)-C(17)-C(13) | 121.2(3) | C(16)-C(17)-C(13) | 103.0(2) |
| C(22)-C(20)-C(17) | 115.0(3) | C(22)-C(20)-C(21) | 109.6(3) |
| C(17)-C(20)-C(21) | 110.8(3) | C(23)-C(22)-C(20) | 115.7(4) |

**Table 4. Anisotropic displacement parameters (Å² x 10³) for 2-MbisP. The anisotropic displacement factor exponent takes the form: -2π²[h² a*² U11 + ... + 2 h k a* b* U12]**

| | U11 | U22 | U33 | U23 | U13 | U12 |
|---|---|---|---|---|---|---|
| O(1) | 36(1) | 59(1) | 92(2) | -7(1) | -3(1) | -5(1) |
| O(3) | 42(1) | 48(1) | 145(2) | 3(1) | -17(2) | 2(1) |
| C(1) | 34(2) | 45(2) | 70(2) | -5(2) | -11(2) | -1(1) |
| C(2) | 39(2) | 51(2) | 83(2) | -21(2) | -14(2) | 6(1) |
| C(3) | 55(2) | 52(2) | 75(2) | -19(2) | -7(2) | 8(2) |
| C(4) | 60(2) | 56(2) | 61(2) | -2(2) | 2(2) | 0(2) |
| C(5) | 52(2) | 34(1) | 58(2) | 3(1) | -5(2) | 1(1) |
| C(6) | 51(2) | 45(2) | 61(2) | 2(1) | 5(2) | -9(2) |
| C(7) | 56(2) | 42(2) | 62(2) | 2(1) | 6(2) | -8(2) |
| C(8) | 47(2) | 44(2) | 58(2) | 3(1) | -1(2) | -10(2) |
| C(9) | 48(2) | 75(2) | 73(2) | -15(2) | 3(2) | -12(2) |
| C(10) | 44(2) | 41(2) | 66(2) | -11(1) | -6(2) | 4(1) |
| C(11) | 59(2) | 78(2) | 72(2) | -12(2) | 11(2) | -32(2) |
| C(12) | 59(2) | 61(2) | 63(2) | -5(2) | 1(2) | -22(2) |
| C(13) | 43(2) | 51(2) | 47(2) | 5(1) | -6(2) | -7(2) |
| C(14) | 45(2) | 52(2) | 53(2) | 4(1) | -4(2) | -8(2) |
| C(15) | 53(2) | 64(2) | 69(2) | 1(2) | 9(2) | -19(2) |
| C(16) | 48(2) | 71(2) | 58(2) | 3(2) | 4(2) | 0(2) |
| C(17) | 49(2) | 53(2) | 50(2) | 1(1) | -7(2) | -1(2) |
| C(18) | 71(3) | 66(2) | 57(2) | 10(2) | -11(2) | -5(2) |
| C(19) | 53(3) | 122(4) | 117(3) | -68(3) | -9(3) | -8(2) |
| C(20) | 73 (3) | 59(2) | 64(2) | -5(2) | -2(2) | 8(2) |
| C (21) | 117(4) | 82(3) | 76(2) | -16(2) | 20(3) | 9(3) |
| C(22) | 104(4) | 63(2) | 99(3) | -12(2) | 18(3) | -6(2) |
| C(23) | 107(4) | 101(4) | 140(5) | -39(3) | -4(4) | -25(3) |

**Table 5. Hydrogen coordinates (x 10⁴) and isotropic displacement parameters (Å² x 10³) for 2-MbisP.**

| | x | y | z | U(eq) |
|---|---|---|---|---|
| H(10) | 2030 | 2820 | 5188 | 75 |
| H(30) | 9248 | 3381 | 4573 | 94 |
| H(1) | 4744 | 3489 | 5201 | 60 |
| H(3) | 6972 | 3366 | 3580 | 73 |
| H(4A) | 8510 | 4644 | 4021 | 71 |
| H(4B) | 6418 | 4808 | 3682 | 71 |
| H(6) | 8807 | 5506 | 4904 | 63 |
| H(7) | 5716 | 5796 | 5840 | 64 |
| H(9A) | 10678 | 6347 | 5395 | 78 |
| H(9B) | 11311 | 6330 | 6144 | 78 |
| H(10A) | 3261 | 4960 | 4528 | 61 |
| H(10B) | 3817 | 4933 | 5284 | 61 |
| H(11A) | 9979 | 7806 | 5444 | 84 |
| H(11B) | 12157 | 7673 | 5728 | 84 |
| H(12A) | 10992 | 7820 | 6807 | 74 |
| H(12B) | 10264 | 8624 | 6403 | 74 |
| H(14) | 8690 | 6614 | 6986 | 60 |
| H(15A) | 5627 | 6117 | 7210 | 74 |
| H(15B) | 4592 | 6682 | 6655 | 74 |
| H(16A) | 5482 | 7201 | 7925 | 71 |
| H(16B) | 4186 | 7714 | 7405 | 71 |
| H(17) | 8337 | 7857 | 7652 | 61 |
| H(18A) | 6780 | 8804 | 6077 | 97 |
| H(18B) | 5109 | 8148 | 6295 | 97 |
| H(18C) | 6593 | 7911 | 5719 | 97 |
| H(19A) | 3904 | 2689 | 3412 | 117 |
| H(19B) | 2083 | 2780 | 3935 | 117 |
| H(20) | 5547 | 9133 | 7268 | 78 |
| H(21A) | 7037 | 8779 | 8551 | 138 |
| H(21B) | 4785 | 8697 | 8323 | 138 |
| H(21C). | 5783 | 9609 | 8388 | 138 |
| H(22A) | 8703 | 9627 | 6950 | 106. |
| H(22B) | 7832 | 10203 | 7518 | 106 |
| H(23A) | 9974 | 9502 | 8273 | 174 |
| H(23B) | 11194 | 9978 | 7717 | 174 |
| H(23C) | 10902 | 8976 | 7684 | 174 |

**Table 6. Torsion angles [°] for 2-MbisP.**

| | | | |
|---|---|---|---|
| O(1)-C(1)=C(2)-C(19) | 0.5(5) | C(10)-C(1)-C(2)-C(19) | -122.6(4) |
| O(1)-C(1)-C(2)-C(3) | 179.9(3) | C(10)-C(1)-C(2)-C(3) | 56.8(3) |
| C(19)-C(2)-C(3)-O(3) | -111.3 (4) | C(1)-C(2)-C(3)-O(3) | 69.2(3) |
| C(19)-C(2)-C(3)-C(4) | 125.8(4) | C(1)-C(2)-C(3)-C(4) | -53.7(4) |
| O(3)-C(3)-C(4)-C(5) | -71.4(4) | C(2)-C(3)-C(4)-C(5) | 48.9(4) |
| C(3)-C(4)-C(5)-C(6) | 128.0(3) | C(3)-C(4)-C(5)-C(10) | -50.1(4) |
| C(10)-C(5)-C(6)-C(7) | -2.4(5) | C(4)-C(5)-C(6)-C(7) | 179.7(3) |
| C(5)-C(6)-C(7)-C(8) | -178.8(3) | C(6)-C(7)-C(8)-C(9) | 1.4(5) |
| C(6)-C(7)-C(8)-C(14) | -179.6(3) | C(7)-C(8)-C(9)-C(11) | 125.7(3) |
| C(14)-C(8)-C(9)-C(11) | -53.4(4) | C(6)-C(5)-C(10)-C(1) | -125.0(3) |
| C(4)-C(5)-C(10)-C(1) | 53.1(3) | O(1)-C(1)-C(10)-C(5) | 179.9(2) |
| C(2)-C(1)-C(10)-C(5) | -54.7(3) | C(8)-C(9)-C(11)-C(12) | 53.0(4) |
| C(9)-C(11)-C(12)-C(13) | -54.6(4) | C(11)-C(12)-C(13)-C(18) | -66.8(4) |
| C(11)-C(12)-C(13)-C(14) | 54.5(4) | C(11)-C(12)-C(13)-C(17) | 166.4(3) |
| C(7)-C(8)-C(14)-C(15) | 3.9(5) | C(9)-C(8)-C(14)-C(15) | -177.0(3) |
| C(7)-C(8)-C(14)-C(13) | -122.0(3) | C(9)-C(8)-C(14)-C(13) | 57.2(4) |
| C(12)-C(13)-C(14)-C(15) | 168.8(3) | C(18)-C(13)-C(14)-C(15) | -70.1(3) |
| C(17)-C(13)-C(14)-C(15) | 45.6(3) | C(12)-C(13)-C(14)-C(8) | -57.1(3) |
| C(18)-C(13)-C(14)-C(8) | 64.1(4) | C(17)-C(13)-C(14)-C(8) | 179.7(3) |
| C(8)-C(14)-C(15)-C(16) | -163.1(3) | C(13)-C(14)-C(15)-C(16) | -33.1(3) |
| C(14)-C(15)-C(16)-C(17) | 7.4(4) | C(15)-C(16)-C(17)-C(20) | 151.9(3) |
| C(15)-C(16)-C(17)-C(13) | 20.6(3) | C(12)-C(13)-C(17)-C(20) | 79.2(4) |
| C(18)-C(13)-C(17)-C(20) | -48.2(4) | C(14)-C(13)-C(17)-C(20) | -164.7(3) |
| C(12)-C(13)-C(17)-C(16) | -155.7(3) | C(18)-C(13)-C(17)-C(16) | 76.9(3) |
| C(14)-C(13)-C(17)-C(16) | -39.5(3) | C(16)-C(17)-C(20)-C(22) | -178.0(3) |
| C(13)-C(17)-C(20)-C(22) | -56.8(5) | C(16)-C(17)-C(20)-C(21) | 56.9(4) |
| C(13)-C(17)-C(20)-C(21) | 178.1(3) | C(17)-C(20)-C(22)-C(23) | -62.9(5) |
| C(21)-C(20)-C(22)-C(23) | 62.8(5) | | |

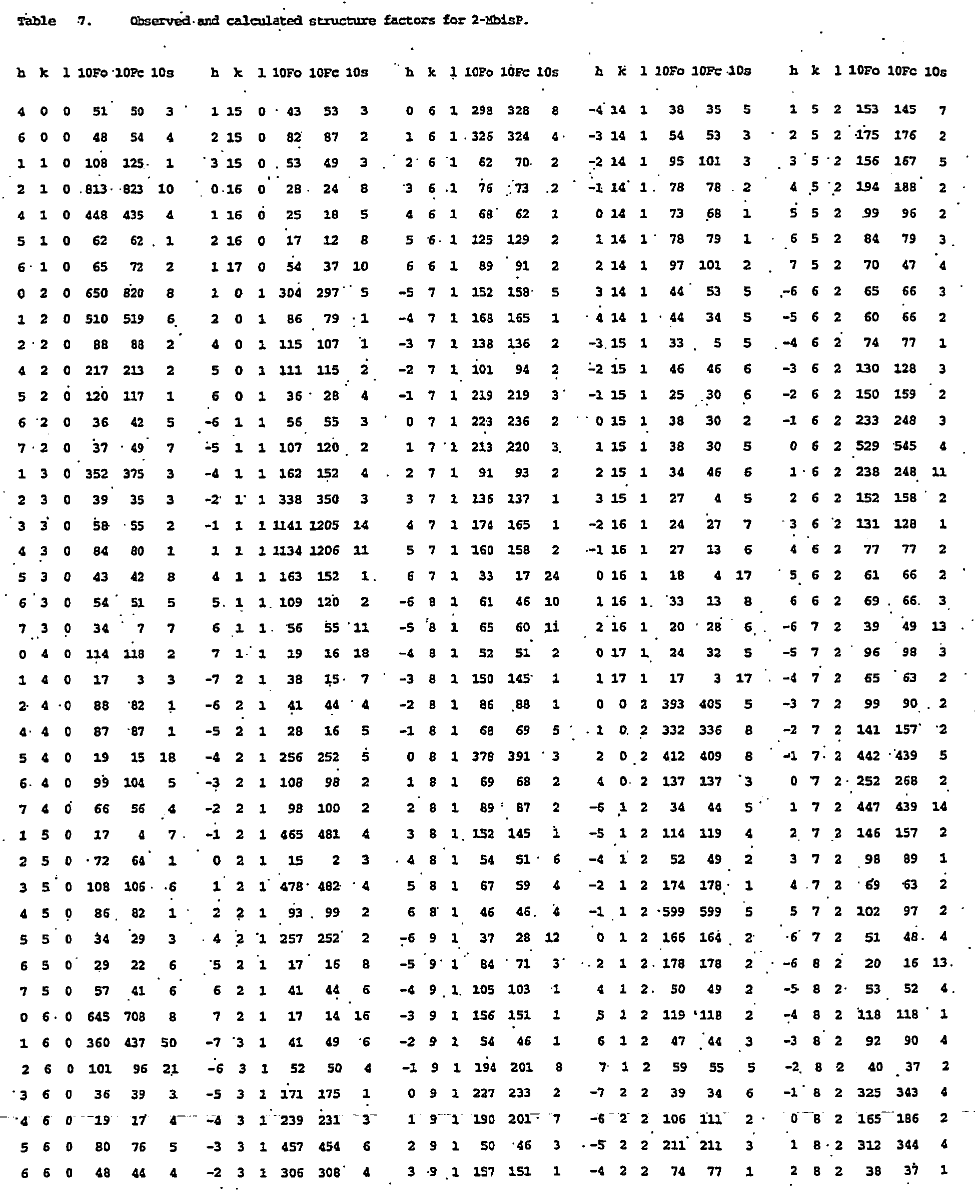

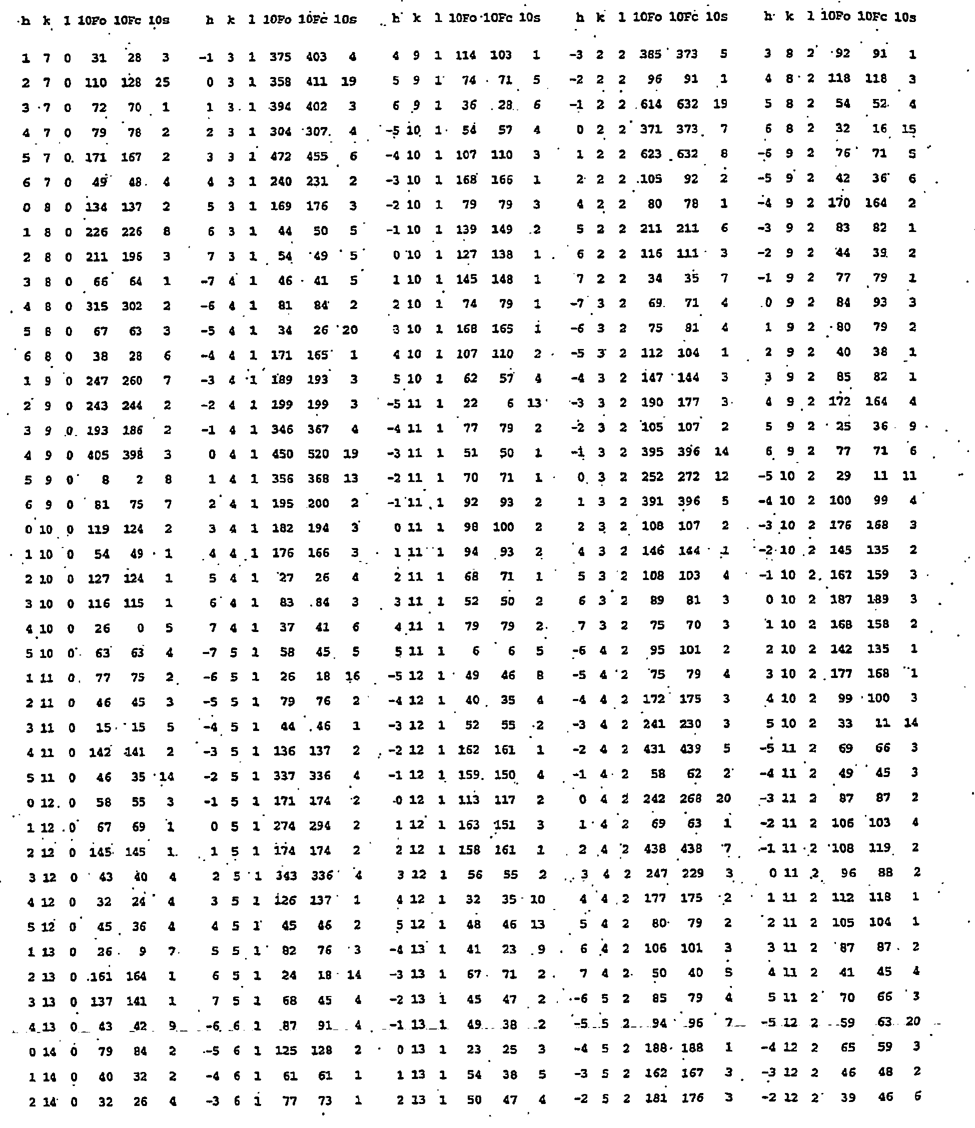

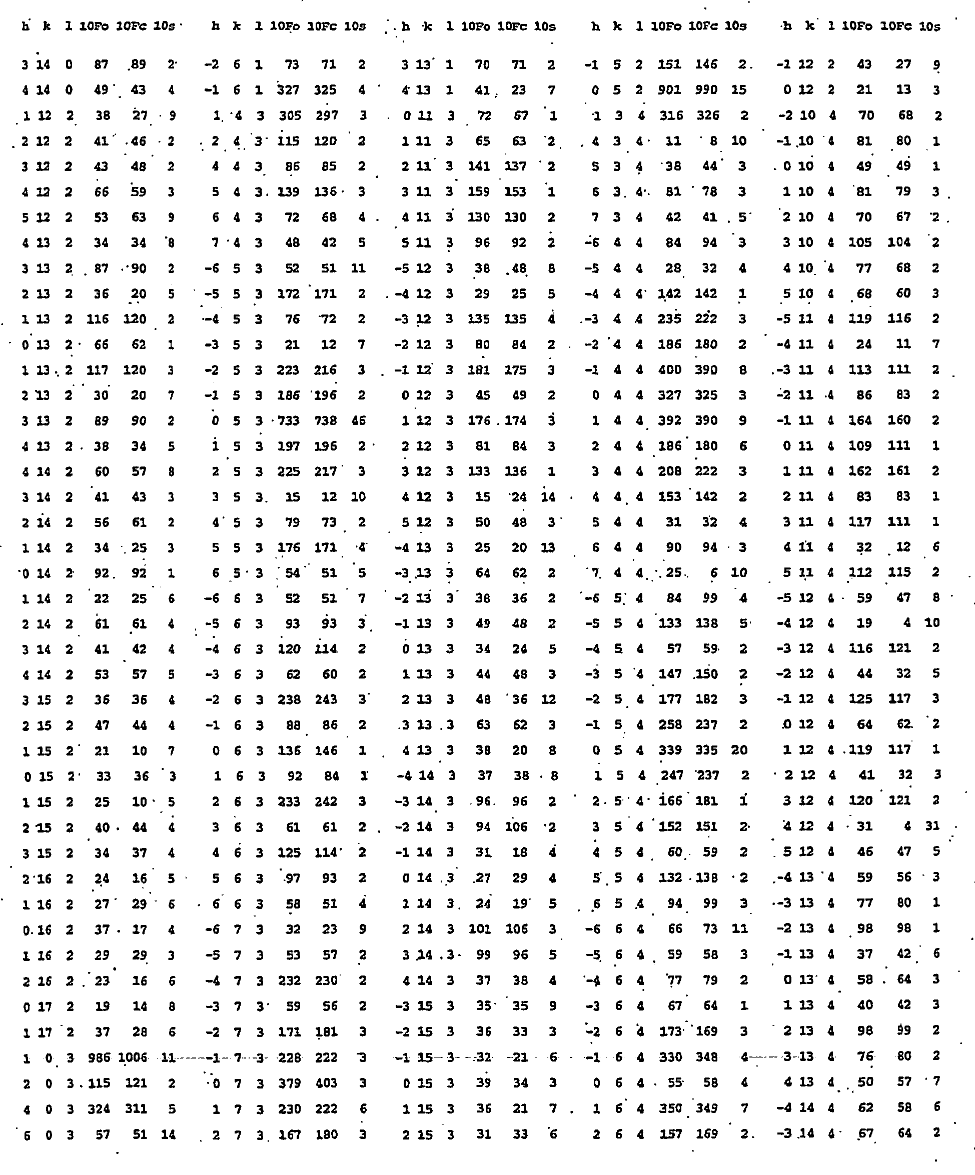

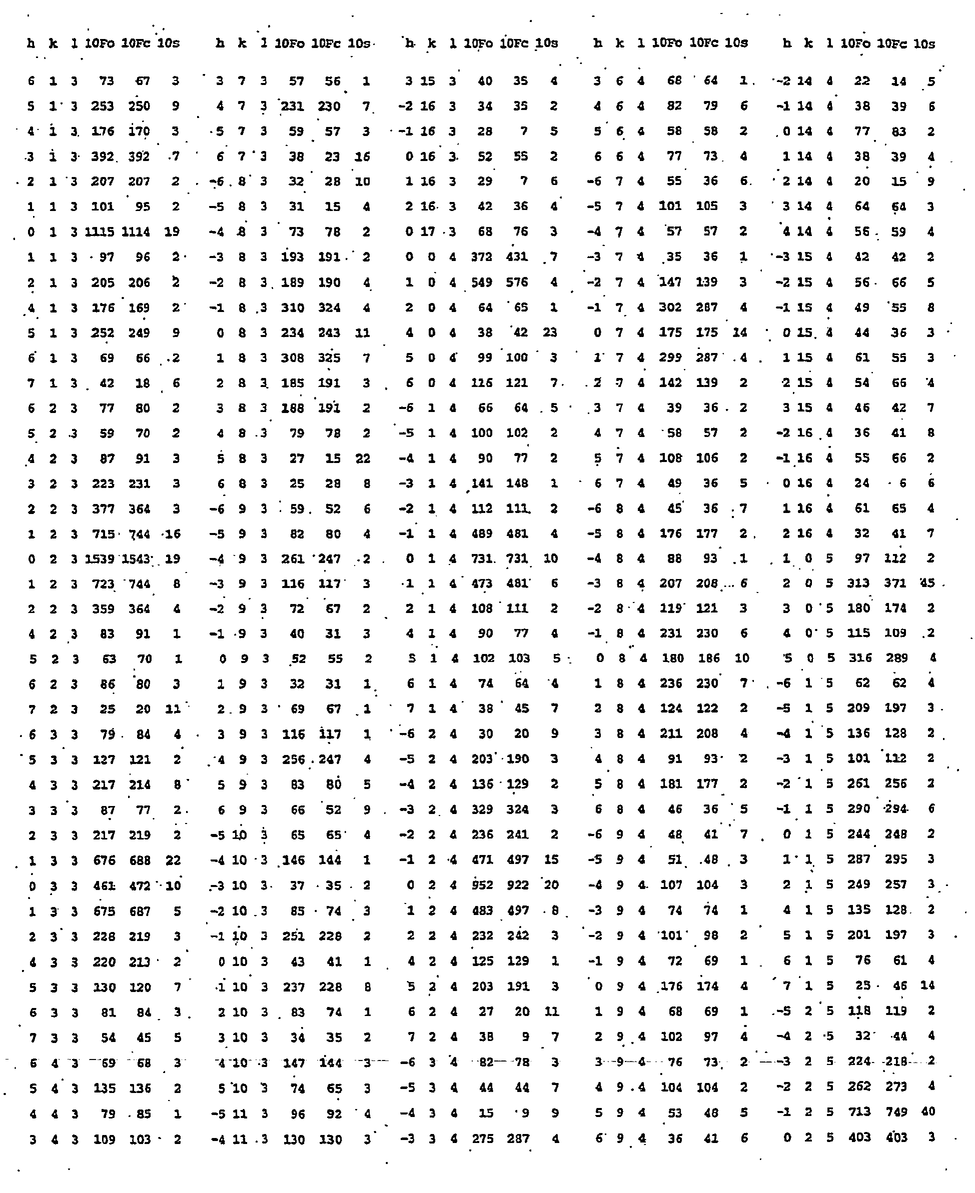

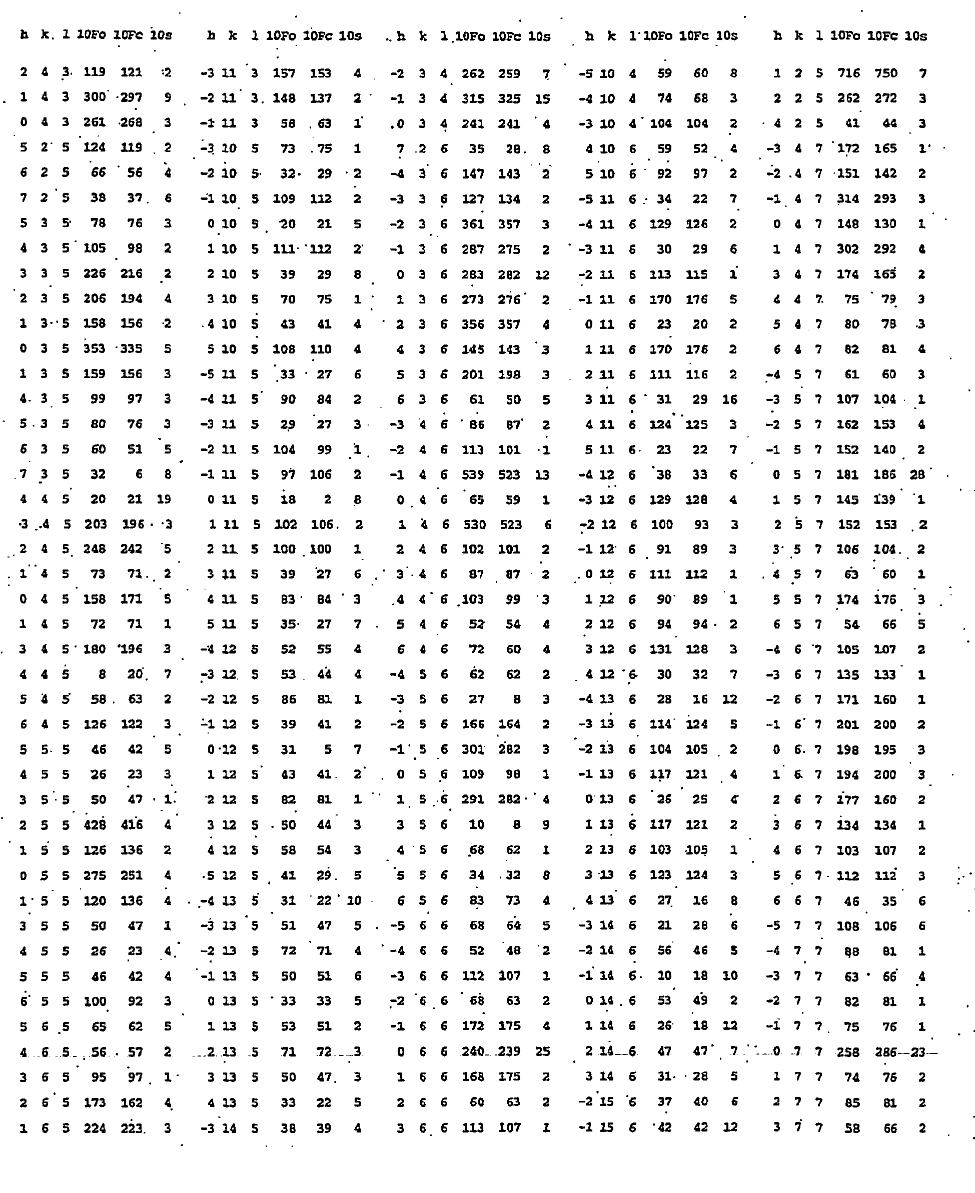

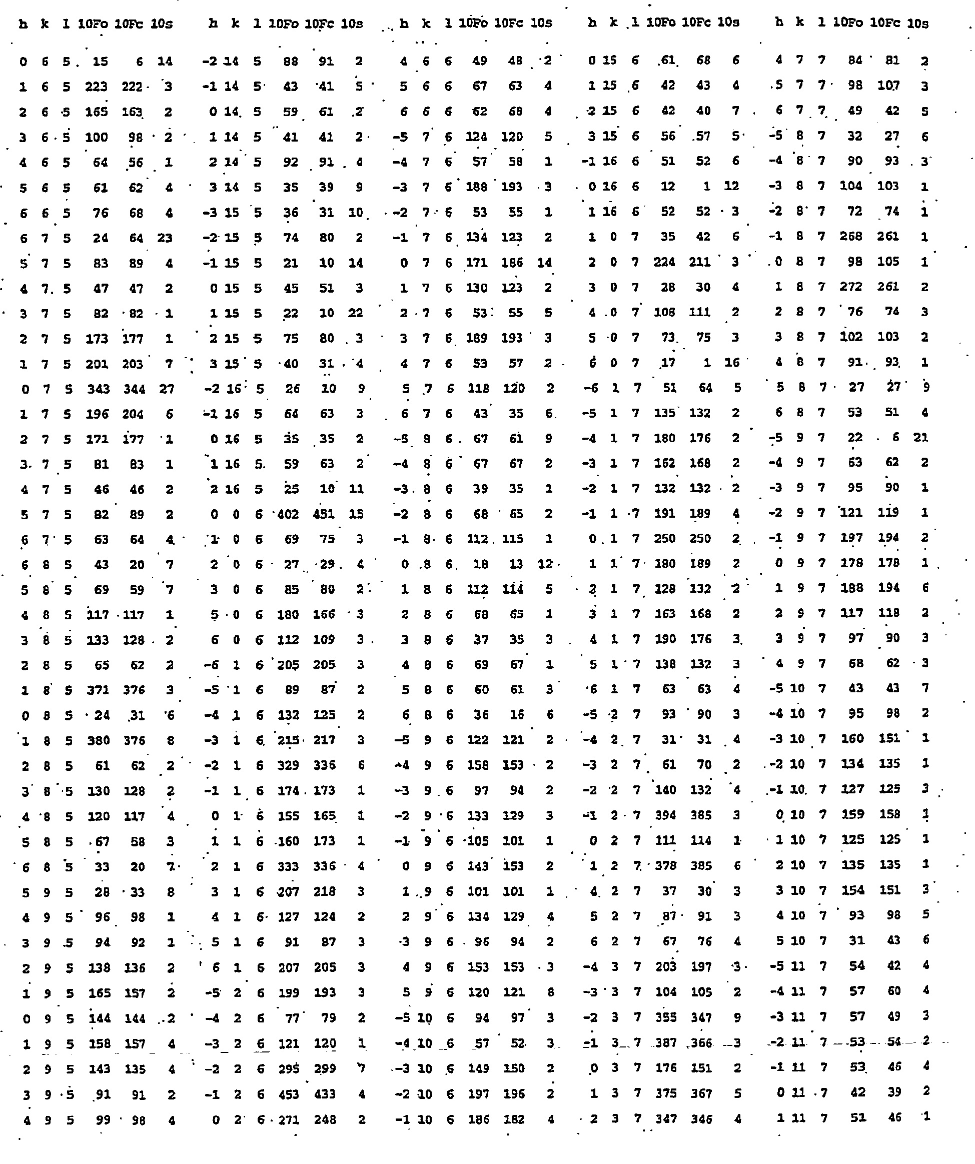

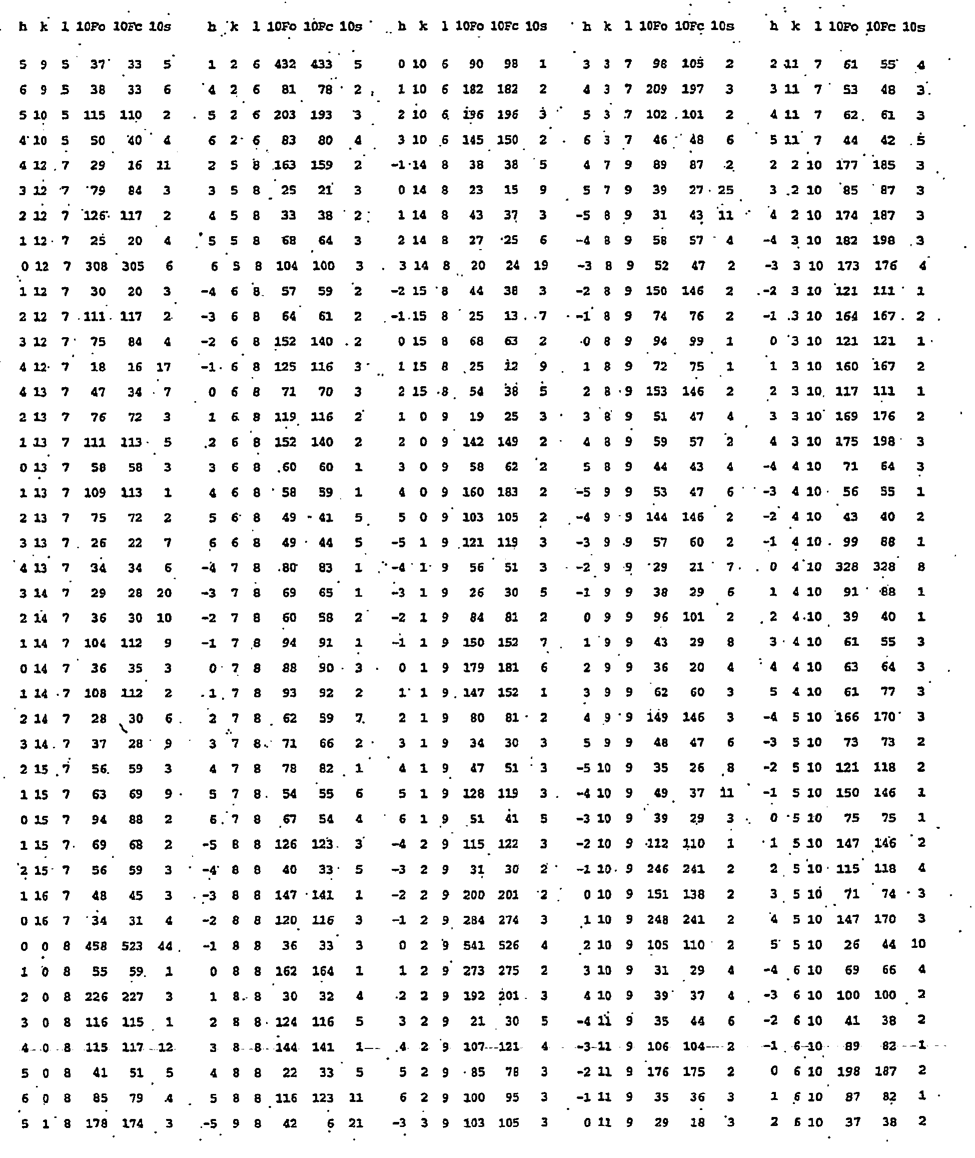

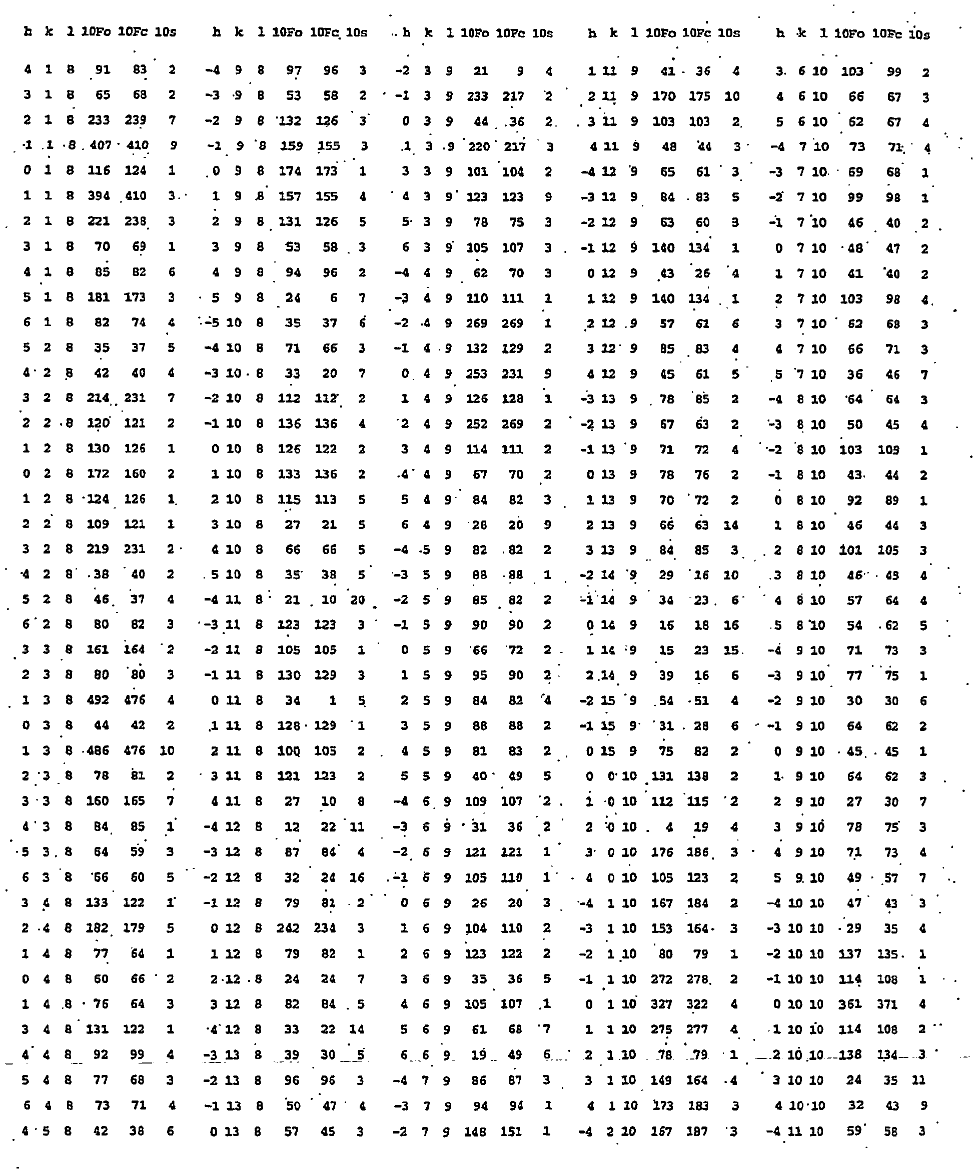

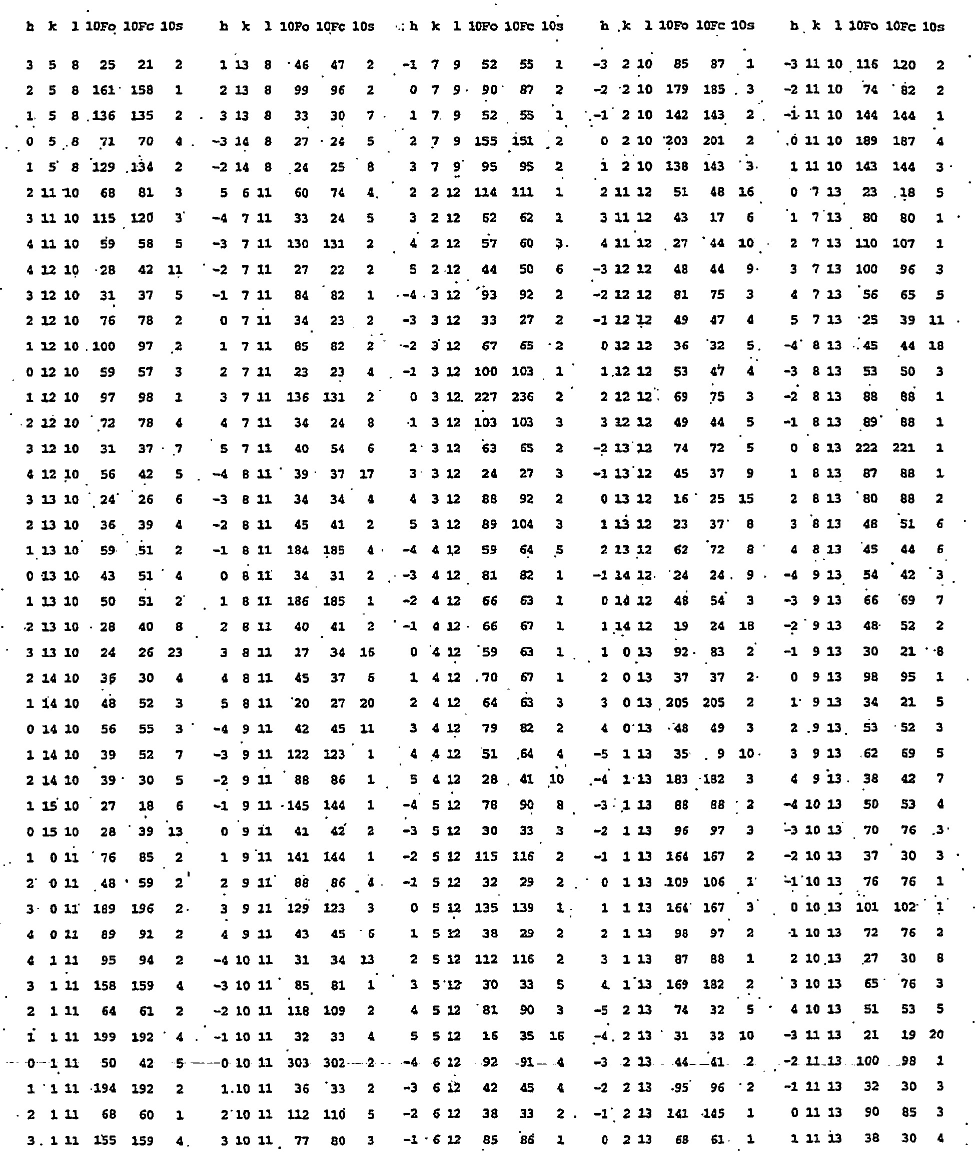

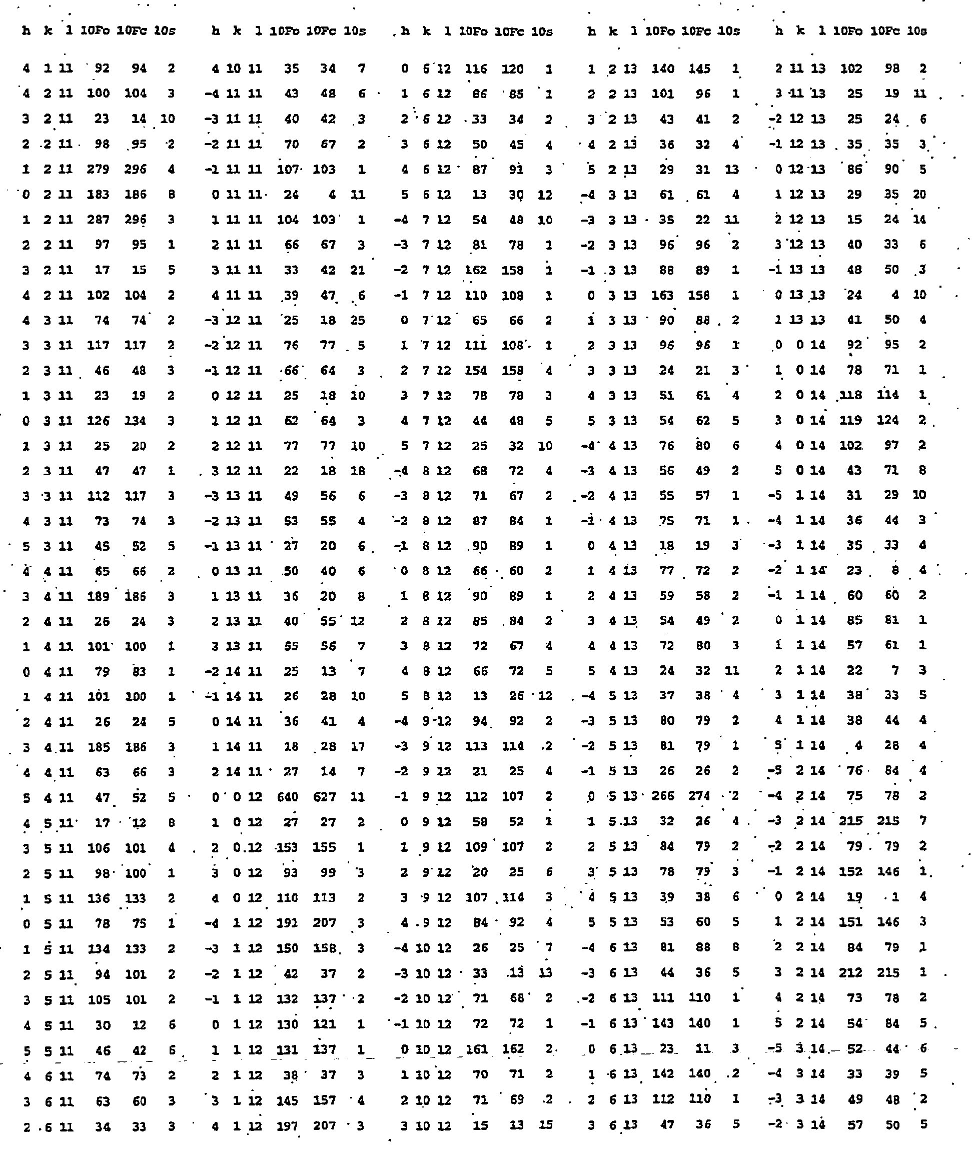

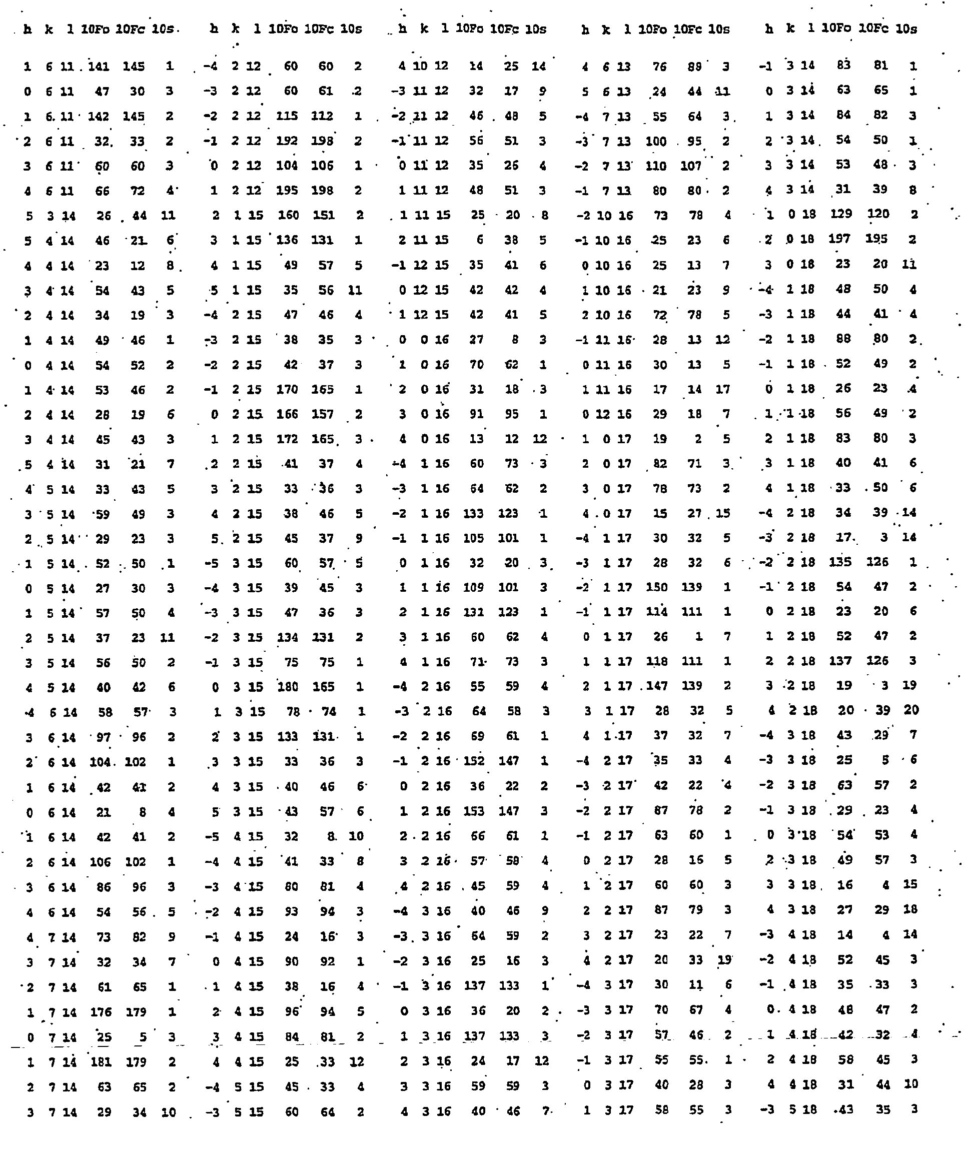

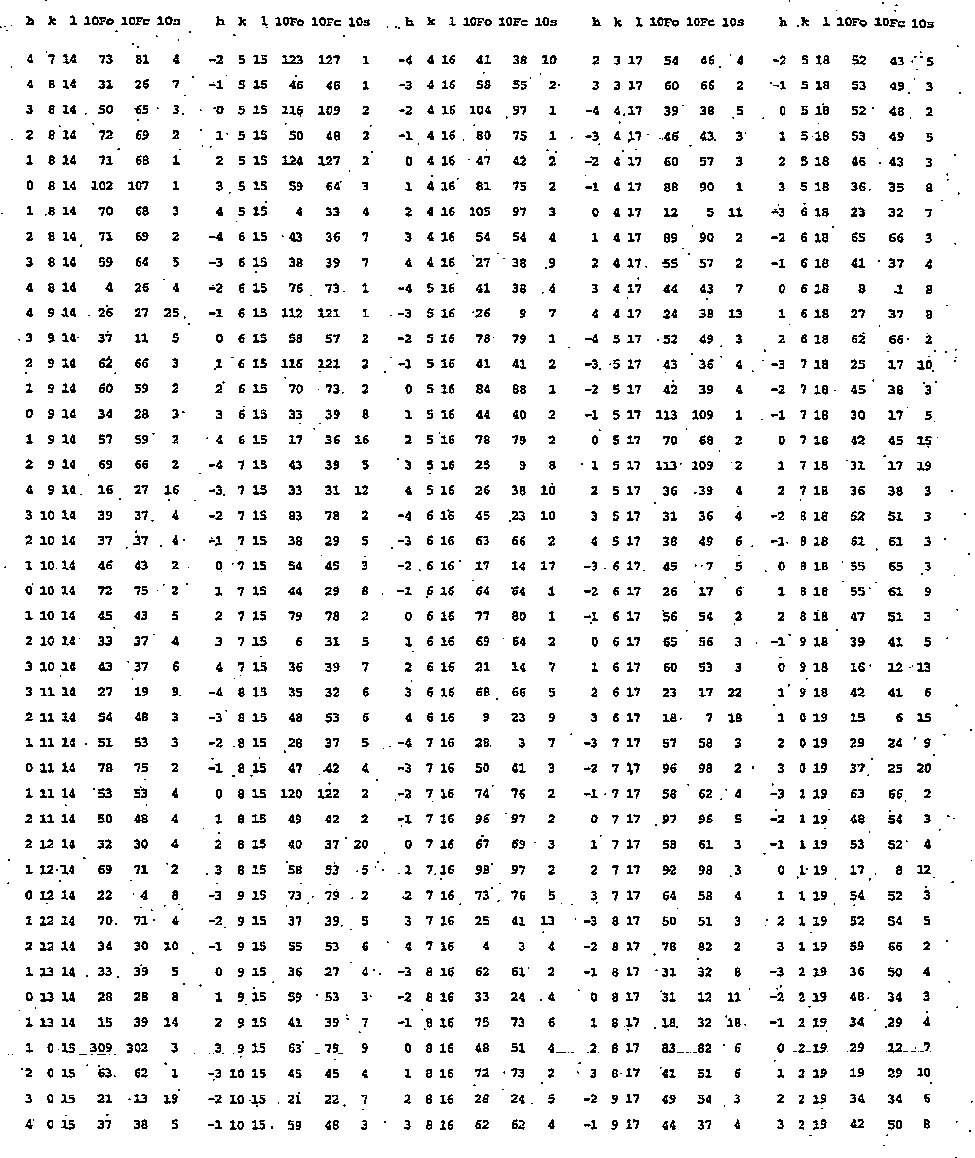

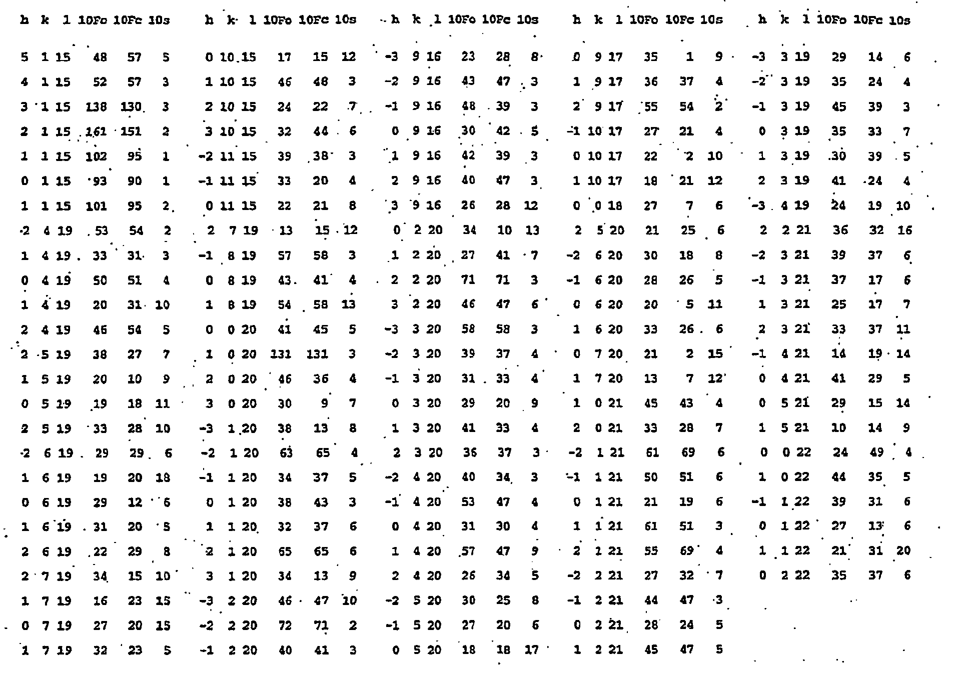

## Claims

1. (20S)-1α-hydroxy-2-methylene-19-nor-bishomopregnacalciferol having the formula:

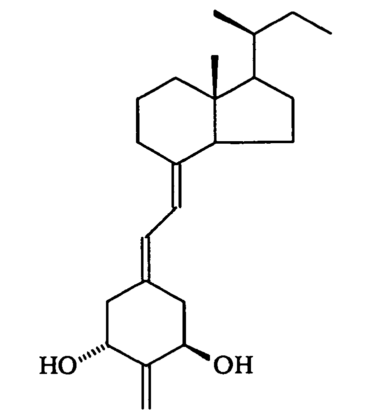

2. (20S)-1α-hydroxy-2-methylene-19-nor-bishomopregnacalciferol in crystalline form.

3. A crystalline form according to claim 2, having a molecular packing arrangement defined by orthorhombic space group P2(1)2(1)2(1) and unit cell dimensions a=6.6A°, b=15.5A°, c=20.1A°, α=90° and γ=90°.

4. Use of an effective amount of (20S)-1α-hydroxy-2-methylene-19-norbishomopregnacalciferol having the formula: 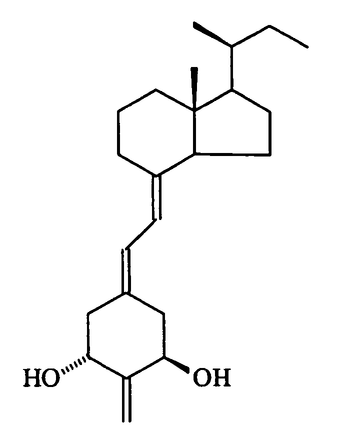 in the preparation of a medicament for the treatment of psoriasis.

5. Use according to claim 4, wherein (20S)-1α-hydroxy-2-methylene-19-nor-bishomopregnacalciferol is suitable for oral, parenteral, transdermal or topical administration.

6. Use according to claim 4, wherein the medicament comprises (20S)-1α-hydroxy-2-methylene-19-nor-bishomopregnacalciferol in an amount of from 0.01 to 100 µg/g of the composition.

7. Use of an effective amount of (20S)-1α-hydroxy-2-methylene-19-norbishomopregnacalciferol having the formula: 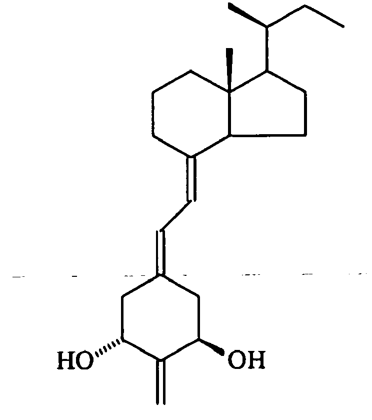 in the preparation of a medicament for the treatment of a disease chosen from leukemia, colon cancer, breast cancer and prostate cancer.

8. Use according to claim 7, wherein (20S)-1α-hydroxy-2-methylene-19-nor-bishomopregnacalciferol is suitable for oral, parenteral or transdermal administration.

9. Use according to claim 7, wherein the medicament comprises (20S)-1α-hydroxy-2-methylene-19-nor-bishomopregnacalciferol in an amount of from 0.01 to 100 µg/g of the composition.

10. Use of an effective amount of (20S)-1α-hydroxy-2-methylene-19-norbishomopregnacalciferol having the formula: 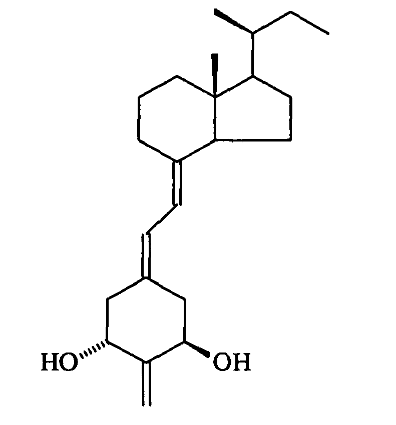 in the preparation of a medicament for the treatment of an autoimmune disease chosen from multiple sclerosis, lupus, diabetes mellitus, host versus graft reaction and rejection of organ transplants.

11. Use according to claim 10, wherein (20S)-1α-hydroxy-2-methylene-19-nor-bishomopregnacalciferol is suitable for oral, parenteral or transdermal administration.

12. Use according to claim 10, wherein the medicament comprises (20S)-1α-hydroxy-2-methylene-19-nor-bishomopregnacalciferol in an amount of from 0.01 to 100 µg/g of the composition.

13. Use of an effective amount of (20S)-1α-hydroxy-2-methylene-19-norbishomopregnacalciferol having the formula: 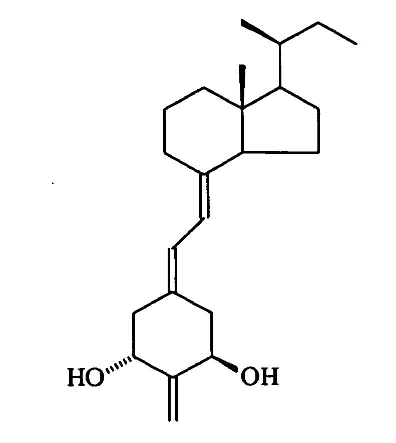 in the preparation of a medicament for the treatment of an inflammatory disease chosen from rheumatoid arthritis, asthma and inflammatory bowel diseases.

14. Use according to claim 13, wherein (20S)-1α-hydroxy-2-methylene-19-nor-bishomopregnacalciferol is suitable for oral, parenteral or transdermal administration.

15. Use according to claim 13, wherein the medicament comprises (20S)-1α-hydroxy-2-methylene-19-nor-bishomopregnacalciferol in an amount of from 0.01 to 100 µg/g of the composition.

16. Use of an effective amount of (20S)-1α-hydroxy-2-methylene-19-norbishomopregnacalciferol having the formula: 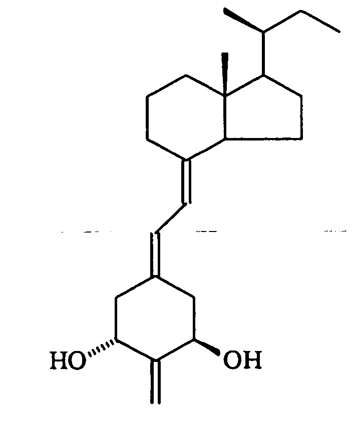 in the preparation of a medicament for the treatment of a skin condition chosen from wrinkles, lack of adequate skin firmness, lack of adequate dermal hydration and insufficient sebum secretion.

17. Use according to claim 16, wherein (20S)-1α-hydroxy-2-methylene-19-nor-bishomopregnacalciferol is suitable for oral, parenteral, transdermal or topical administration.

18. Use according to claim 16, wherein the medicament comprises (20S)-1α-hydroxy-2-methylene-19-nor-bishomopregnacalciferol in an amount of from 0.01 to 100 µg/g of the composition.

19. Use of an effective amount of (20S)-1α-hydroxy-2-methylene-19-norbishomopregnacalciferol having the formula : 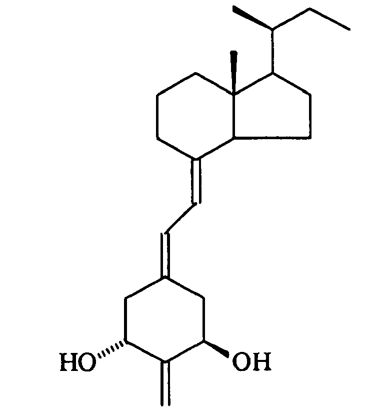 in the preparation of a medicament for the treatment of renal osteodystrophy.

20. Use according to claim 19, wherein (20S)-1α-hydroxy-2-methylene-19-nor-bishomopregnacalciferol is suitable for oral, parenteral or transdermal administration.

21. Use according to claim 19, wherein the medicament comprises (20S)-1α-hydroxy-2-methylene-19-nor-bishomopregnacalciferol in an amount of from 0.01 to 100 µg/g of the composition.

22. A method of purifying (20S)-1α-hydroxy-2-methylene-19-norbishomopregnacalciferol, comprising the steps of :
(a) boiling a solvent chosen from acetone, ethyl acetate, isopropanol, chloroform, dichloromethane and diethyl ether under inert atmosphere;
(b) dissolving a product comprising (20S)-1α-hydroxy-2-methylene-19-nor-bishomopregnacalciferol to be purified in the solvent;
(c) cooling the solvent and dissolved product to below ambient temperature for a sufficient amount of time to form a precipitate of (20S)-1α-hydroxy-2-methylene-19-nor-bishomopregnacalciferol crystals, and
(d) recovering the (20S)-1α-hydroxy-2-methylene-19-norbishomopregnacalciferol crystals.

23. A method according to claim 22, comprising the further step of allowing the solvent and dissolved product to cool to ambient temperature prior to cooling to below ambient temperature.

24. A method according to claim 22, wherein the inert atmosphere is an argon atmosphere.

25. A method according to claim 22, wherein the solvent and dissolved product are cooled to between 1.6 and 7.2 °C (35 to 45 °F).

26. A method according to claim 22, wherein the step of recovering comprises filtering.

27. A method according to claim 22, comprising the further step (e) which comprises repeating steps (a) to (d), using the recovered crystals from step (d) as the product of step (b).

28. (20S)-1α-hydroxy-2-methylene-19-nor-bishomopregnacalciferol having the formula : 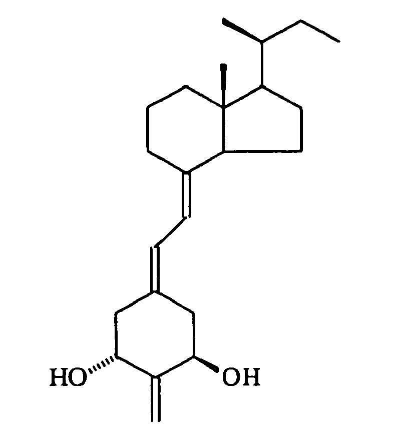 for use in the treatment of
(i) psoriasis;
(ii) a disease chosen from leukemia, colon cancer, breast cancer and prostate cancer;
(iii) an inflammatory disease chosen from rheumatoid arthritis, asthma and inflammatory bowel diseases;
(iv) a skin condition chosen from wrinkles, lack of adequate skin firmness, lack of adequate dermal hydration and insufficient sebum secretion; or
(v) renal osteodystrophy.

## Patentansprüche

1. (20S)-1α-Hydroxy-2-methylen-19-nor-bishomopregnacalciferol der Formel

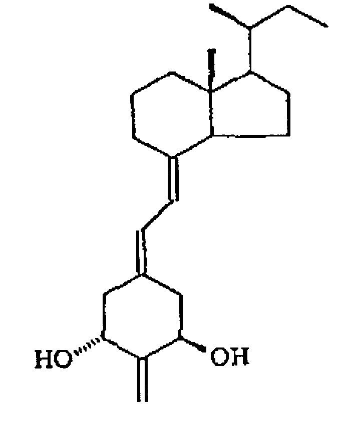

2. (20S)-1α-Hydroxy-2-methylen-19-nor-bishomopregnacalciferol in kristalliner Form.

3. Kristalline Form nach Anspruch 2 mit einer molekularen Packungsanordnung, die durch die orthorhombische Raumgruppe P2(1)2(1)2(1) und Elementarzellabmessungen a=6,6 Å, b=15,5 Å, c=20,1 Å, α=90° und γ=90° definiert ist.

4. Verwendung einer wirksamen Menge von (20S)-1α-Hydroxy-2-methylen-19-nor-bishomopregnacalciferol der Formel 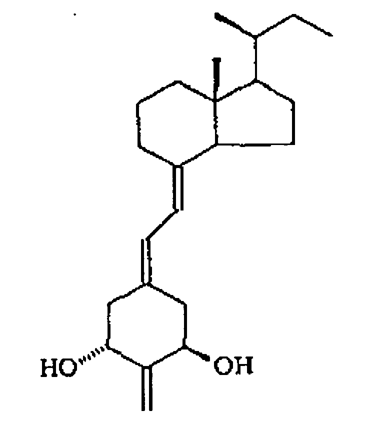 bei der Herstellung eines Arzneimittels zur Behandlung von Psoriasis.

5. Verwendung nach Anspruch 4, wobei das (20S)-1α-Hydroxy-2-methylen-19-nor-bishomopregnacalciferol für die orale, parenterale, transdermale oder topische Verabreichung geeignet ist.

6. Verwendung nach Anspruch 4, wobei das Arzneimittel (20S)-1α-Hydroxy-2-methylen-19-nor-bishomopregnacalciferol in einer Menge von 0,01 bis 100 µg/g der Zusammensetzung umfasst.

7. Verwendung einer wirksamen Menge von (20S)-1α-Hydroxy-2-methylen-19-nor-bishomopregnacalciferol der Formel 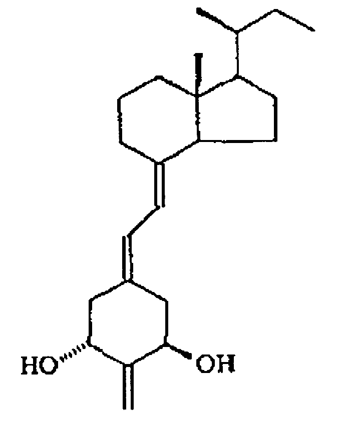 bei der Herstellung eines Arzneimittels zur Behandlung einer Krankheit, die aus Leukämie, Kolonkrebs, Brustkrebs und Prostatakrebs ausgewählt ist.

8. Verwendung nach Anspruch 7, wobei das (20S)-1α-Hydroxy-2-methylen-19-nor-bishomopregnacalciferol für die orale, parenterale oder transdermale Verabreichung geeignet ist.

9. Verwendung nach Anspruch 7, wobei das Arzneimittel (20S)-1α-Hydroxy-2-methylen-19-nor-bishomopregnacalciferol in einer Menge von 0,01 bis 100 µg/g der Zusammensetzung umfasst.

10. Verwendung einer wirksamen Menge von (20S)-1α-Hydroxy-2-methylen-19-nor-bishomopregnacalciferol der Formel 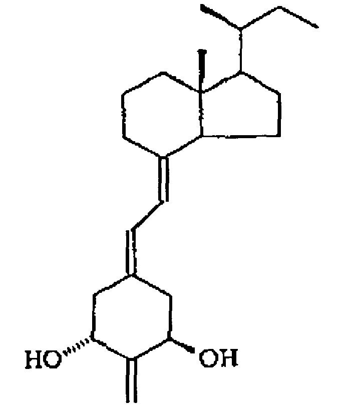 bei der Herstellung eines Arzneimittels zur Behandlung einer Autoimmunkrankheit, die aus multipler Sklerose, Lupus, Diabetes mellitus, Host-versus-Graft-Reaktion und Abstoßung von Organtransplantaten ausgewählt ist.

11. Verwendung nach Anspruch 10, wobei das (20S)-1α-Hydroxy-2-methylen-19-nor-bishomopregnacalciferol für die orale, parenterale oder transdermale Verabreichung geeignet ist.

12. Verwendung nach Anspruch 10, wobei das Arzneimittel (20S)-1α-Hydroxy-2-methylen-19-nor-bishomopregnacalciferol in einer Menge von 0,01 bis 100 µg/g der Zusammensetzung umfasst.

13. Verwendung einer wirksamen Menge von (20S)-1α-Hydroxy-2-methylen-19-nor-bishomopregnacalciferol der Formel 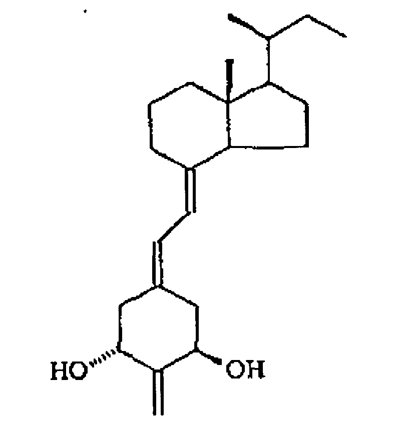 bei der Herstellung eines Arzneimittels zur Behandlung einer entzündlichen Krankheit, die aus rheumatoider Arthritis, Asthma und entzündlichen Darmkrankheiten ausgewählt ist.

14. Verwendung nach Anspruch 13, wobei das (20S)-1α-Hydroxy-2-methylen-19-nor-bishomopregnacalciferol für die orale, parenterale oder transdermale Verabreichung geeignet ist.

15. Verwendung nach Anspruch 13, wobei das Arzneimittel (20S)-1α-Hydroxy-2-methylen-19-nor-bishomopregnacalciferol in einer Menge von 0,01 bis 100 µg/g der Zusammensetzung umfasst.

16. Verwendung einer wirksamen Menge von (20S)-1α-Hydroxy-2-methylen-19-nor-bishomopregnacalciferol der Formel 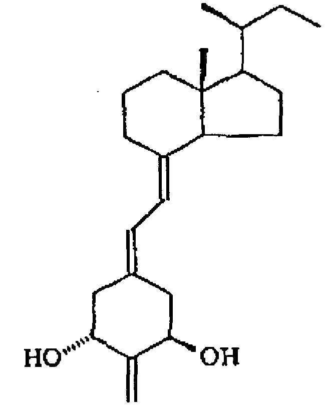 bei der Herstellung eines Arzneimittels zur Behandlung eines Hautzustands, der aus Falten, Mangel an angemessener Hautfestigkeit, Mangel an angemessener dermaler Hydratisierung und unzureichender Sebumsekretion ausgewählt ist.

17. Verwendung nach Anspruch 16, wobei das (20S)-1α-Hydroxy-2-methylen-19-nor-bishomopregnacalciferol für die orale, parenterale, transdermale oder topische Verabreichung geeignet ist.

18. Verwendung nach Anspruch 16, wobei das Arzneimittel (20S)-1α-Hydroxy-2-methylen-19-nor-bishomopregnacalciferol in einer Menge von 0,01 bis 100 µg/g der Zusammensetzung umfasst.

19. Verwendung einer wirksamen Menge von (20S)-1α-Hydroxy-2-methylen-19-nor-bishomopregnacalciferol der Formel 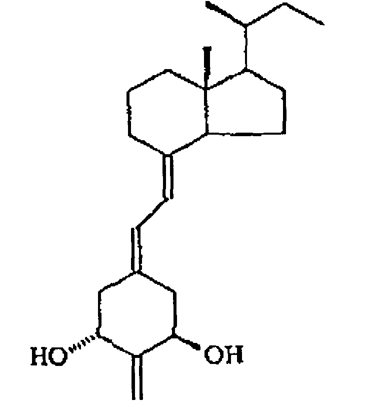 bei der Herstellung eines Arzneimittels zur Behandlung von renaler Osteodystrophie.

20. Verwendung nach Anspruch 19, wobei das (20S)-1α-Hydroxy-2-methylen-19-nor-bishomopregnacalciferol für die orale, parenterale oder transdermale Verabreichung geeignet ist.

21. Verwendung nach Anspruch 19, wobei das Arzneimittel (20S)-1α-Hydroxy-2-methylen-19-nor-bishomopregnacalciferol in einer Menge von 0,01 bis 100 µg/g der Zusammensetzung umfasst.

22. Verfahren zur Reinigung von (20S)-1α-Hydroxy-2-methylen-19-nor-bishomopregnacalciferol, umfassend die folgenden Stufen
(a) das Erhitzen eines Lösungsmittels, das aus Aceton, Ethylacetat, Isopropanol, Chloroform, Dichlormethan und Diethylether ausgewählt ist, unter einer inerten Atmosphäre auf die Siedetemperatur;
(b) das Lösen eines Produkts, das zu reinigendes (20S)-1α-Hydroxy-2-methylen-19-nor-bishomopregnacalciferol umfasst, im Lösungsmittel;
(c) das Abkühlen des Lösungsmittels und des gelösten Produkts auf eine Temperatur unter Umgebungstemperatur für eine ausreichende Zeitspanne, um einen Niederschlag von (20S)-1α-Hydroxy-2-methylen-19-norbishomopregnacalciferol-Kristallen zu bilden, und
(d) das Gewinnen der (20S)-1α-Hydroxy-2-methylen-19-norbishomopregnacalciferol-Kristalle.

23. Verfahren nach Anspruch 22, umfassend die zusätzliche Stufe, bei der man das Lösungsmittel und das gelöste Produkt auf Umgebungstemperatur abkühlen lässt, bevor man die Abkühlung auf eine Temperatur unter Umgebungstemperatur vornimmt.

24. Verfahren nach Anspruch 22, wobei es sich bei der inerten Atmosphäre um eine Argonatmosphäre handelt.

25. Verfahren nach Anspruch 22, wobei das Lösungsmittel und das gelöste Produkt auf 1,6 bis 7,2 °C (35 bis 45 °F) abgekühlt werden.

26. Verfahren nach Anspruch 22, wobei die Gewinnungsstufe eine Filtration umfasst.

27. Verfahren nach Anspruch 22, umfassend die zusätzliche Stufe (e), die die Wiederholung der Stufen (a) bis (d) umfasst, wobei die in Stufe (d) gewonnenen Kristalle als das Produkt von Stufe (b) verwendet werden.

28. (20S)-1α-Hydroxy-2-methylen-19-nor-bishomopregnacalciferol der Formel 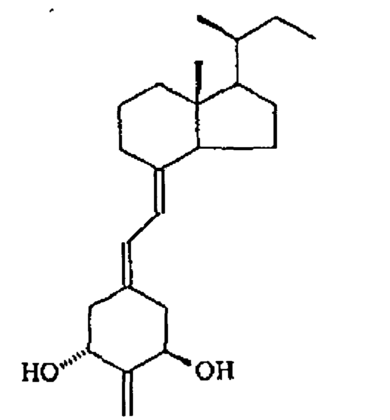 zur Behandlung von
(i) Psoriasis;
(ii) einer Krankheit, die aus Leukämie, Kolonkrebs, Brustkrebs und Prostatakrebs ausgewählt ist;
(iii) einer entzündlichen Krankheit, die aus rheumatoider Arthritis, Asthma und entzündlichen Darmkrankheiten ausgewählt ist;
(iv) einem Hautzustand, der aus Falten, Mangel an angemessener Hautfestigkeit, Mangel an angemessener dermaler Hydratisierung und unzureichender Sebumsekretion ausgewählt ist; oder
(v) renaler Osteodystrophie.

## Revendications

1. (20S)-1α-hydroxy-2-méthylène-19-nor-bishomopregnacalciférol répondant à la formule :

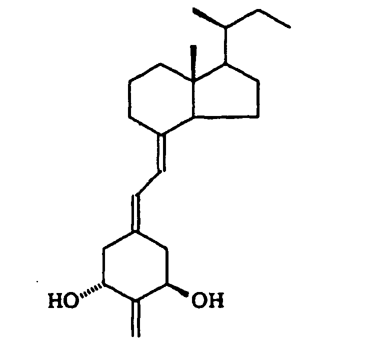

2. (20S)-1α-hydroxy 2-méthylène-19-nor-bishomopregnacalciférol sous forme cristalline.

3. Forme cristalline selon la revendication 2, ayant un arrangement de tassement moléculaire défini par un groupe spatial orthorhombique P2(1)(1)2(1) et des dimensions de cellule unitaire a = 6,6 A°, b = 15,5 A°, c = 20,1 A°, α = 90° et y = 90°.

4. Utilisation d'une quantité efficace de (20S)- 1α- hydroxy- 2-méthylène- 19-nor- bishomopregnacalciférol répondant à la formule : 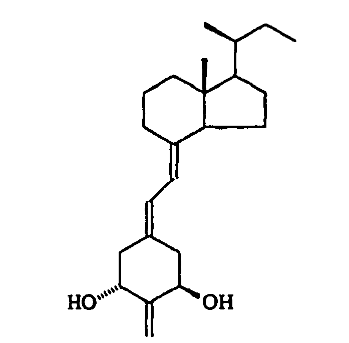 dans la préparation d'un médicament pour le traitement du psoriasis.

5. Utilisation selon la revendication 4, dans laquelle le (20S)- 1α-hydroxy- 2-méthylène- 19-nor- bishomopregnacalciférol convient pour l'administration orale, parentérale, transdermique ou topique.

6. Utilisation selon la revendication 4, dans laquelle le médicament comprend le (20S)- 1α- hydroxy- 2-méthylène- 19-nor- bishomopregnacalciférol en une quantité allant de 0,01 à 100 µg / g de la composition.

7. Utilisation d'une quantité efficace de (20S)- 1α- hydroxy- 2-méthylène- 19-nor- bishomopregnacalciférol répondant à la formule : 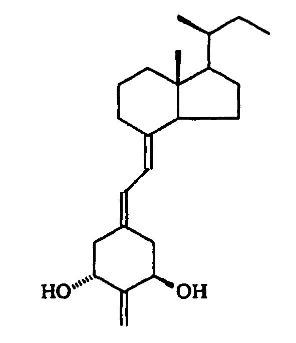 dans la préparation d'un médicament pour le traitement d'une maladie choisie parmi la leucémie, le cancer du côlon, le cancer du sein et le cancer de la prostate.

8. Utilisation selon la revendication 7, dans laquelle le (20S)- 1α-hydroxy- 2-méthylène- 19-nor- bishomopregnacalciférol convient pour l'administration orale, parentérale ou transdermique.

9. Utilisation selon la revendication 7, dans laquelle le médicament comprend le (20S)- 1α- hydroxy- 2-méthylène- 19-nor- bishomopregnacalciférol en une quantité allant de 0,01 à 100 µg / g de la composition.

10. Utilisation d'une quantité efficace de (20S)- 1α- hydroxy- 2-méthylène- 19-nor- bishomopregnacalciférol répondant à la formule : 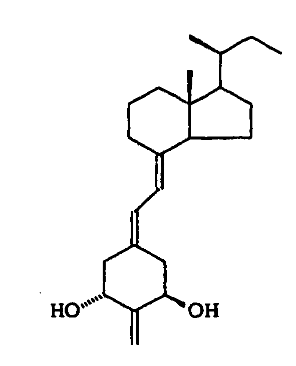 dans la préparation d'un médicament pour le traitement d'une maladie auto-immune choisie parmi la sclérose en plaques, le lupus, le diabète sucré, la réaction hôte contre greffon et le rejet de transplantations d'organes.

11. Utilisation selon la revendication 10, dans laquelle le (20S)- 1α-hydroxy- 2-méthylène- 19-nor- bishomopregnacalciférol convient pour l'administration orale, parentérale ou transdermique.

12. Utilisation selon la revendication 10, dans laquelle le médicament comprend le (20S)- 1α- hydroxy- 2-méthylène- 19-nor- bishomopregnacalciférol en une quantité allant de 0,01 à 100 µg / g de la composition.

13. Utilisation d'une quantité efficace de (20S)- 1α- hydroxy- 2-méthylène- 19-nor- bishomopregnacalciférol répondant à la formule : 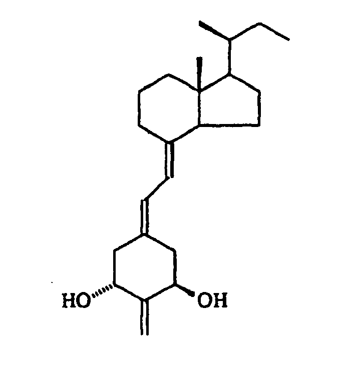 dans la préparation d'un médicament pour le traitement d'une maladie inflammatoire choisie parmi la polyarthrite rhumatoïde, l'asthme et les affections intestinales inflammatoires.

14. Utilisation selon la revendication 13, dans laquelle le (20S)- 1α-hydroxy- 2-méthylène- 19-nor- bishomopregnacalciférol convient pour l'administration orale, parentérale ou transdermique.

15. Utilisation selon la revendication 13, dans laquelle le médicament comprend le (20S)- 1α- hydroxy- 2-méthylène- 19-nor- bishomopregnacalciférol en une quantité allant de 0,01 à 100 µg / g de la composition.

16. Utilisation d'une quantité efficace de (20S)- 1α- hydroxy- 2-méthylène- 19-nor- bishomopregnacalciférol répondant à la formule : 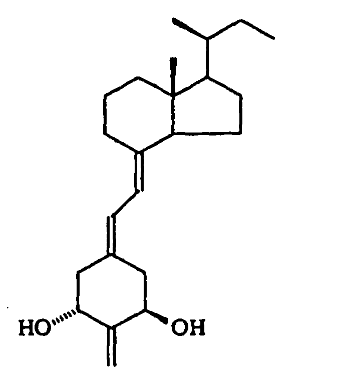 dans la préparation d'un médicament pour le traitement d'une affection de la peau choisie parmi les rides, le manque de fermeté adéquate de la peau, le manque d'hydratation dermique convenable et la sécrétion insuffisante de sébum.

17. Utilisation selon la revendication 16, dans laquelle le (20S)- 1α-hydroxy- 2-méthylène- 19-nor- bishomopregnacalciférol convient pour l'administration orale, parentérale, transdermique ou topique.

18. Utilisation selon la revendication 16, dans laquelle le médicament comprend le (20S)- 1α- hydroxy- 2-méthylène- 19-nor- bishomopregnacalciférol en une quantité allant de 0,01 à 100 µg / g de la composition.

19. Utilisation d'une quantité efficace de (20S)- 1α- hydroxy- 2-méthylène- 19-nor- bishomopregnacalciférol répondant à la formule : 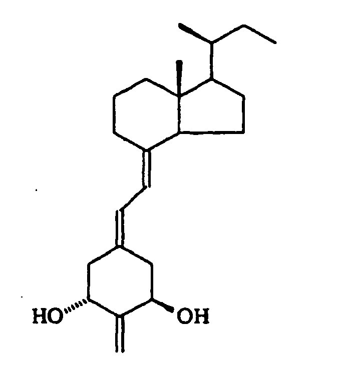 dans la préparation d'un médicament pour le traitement de l'ostéodystrophie rénale.

20. Utilisation selon la revendication 19, dans laquelle le (20S)- 1α-hydroxy- 2-méthylène- 19-nor- bishomopregnacalciférol convient pour l'administration orale, parentérale ou transdermique.

21. Utilisation selon la revendication 19, dans laquelle le médicament comprend le (20S)- 1α- hydroxy- 2-méthylène- 19-nor- bishomopregnacalciférol en une quantité allant de 0,01 à 100 µg / g de la composition.

22. Procédé de purification du (20S)- 1α- hydroxy- 2-méthylène- 19-norbishomopregnacalciférol , qui comprend les étapes qui consistent à :
(a) faire bouillir un solvant choisi parmi l'acétone, l'acétate d'éthyle, l'isopropanol, le chloroforme, le dichlorométhane et l'éther diéthylique sous une atmosphère inerte ;
(b) disoudre un produit comprenant le (20S)- 1α- hydroxy- 2-méthylène-19-nor- bishomopregnacalciférol à purifier dans le solvant ;
(c) refroidir le solvant et le produit dissous au-dessous de la température ambiante pendant un temps suffisant pour former un précipité de cristaux de (20S)- 1α-hydroxy- 2-méthylène- 19-nor- bishomopregnacalciférol , et
(d) récupérer les cristaux de (20S)- 1α- hydroxy- 2-méthylène- 19-norbishomopregnacalciférol .

23. Procédé selon la revendication 22, qui comprend l'étape supplémentaire qui consiste à laisser le solvant et le produit dissous refroidir à la température ambiante avant le refroidissement au-dessous de la température ambiante.

24. Procédé selon la revendication 22, dans lequel l'atmosphère inerte est une atmosphère d'argon.

25. Procédé selon la revendication 22 , dans lequel le solvant et le produit dissous sont refroidis entre 1,6 et 7,2 °C (35 à 45 °F).

26. Procédé selon la revendication 22, dans lequel l'étape de récupération comprend la filtration.

27. Procédé selon la revendication 22, qui comprend l'étape supplémentaire ( e) qui comprend la répétition des étapes (a) à (d), en utilisant les cristaux récupérés de l'étape (d) à titre de produit de l'étape (b).

28. (20S)- 1α- hydroxy- 2-méthylène- 19-nor- bishomopregnacalciférol répondant à la formule : 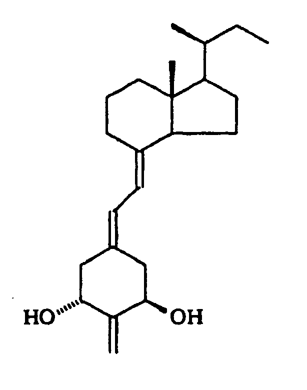 à utiliser dans le traitement :
(i) du psoriasis ;
(ii) d'une maladie choisie parmi la leucémie, le cancer du côlon, le cancer du sein et le cancer de la prostate ;
(iii) d'une maladie inflammatoire choisie parmi la polyarthrite rhumatoïde, l'asthme et les affections intestinales inflammatoires ;
(iv) d'une affection de la peau choisie parmi les rides, le manque de fermeté adéquate de la peau, le manque d'hydratation dermique convenable et la sécrétion insuffisante de sébum ; ou
(v) de l'ostéodystrophie rénale.
